Europäisches Patentamt

European Patent Office

Office européen des brevets

(11) Numéro de publication : **0 396 460 B1**

## (12) FASCICULE DE BREVET EUROPEEN

(45) Date de publication du fascicule du brevet :
07.10.92 Bulletin 92/41

(51) Int. Cl.⁵ : **C01B 33/193, A61K 7/16**

(21) Numéro de dépôt : **90401163.2**

(22) Date de dépôt : **27.04.90**

(54) **Silice pour compositions dentifrices compatible, notamment avec les cations métalliques.**

(30) Priorité : **03.05.89 FR 8905869**

(43) Date de publication de la demande :
**07.11.90 Bulletin 90/45**

(45) Mention de la délivrance du brevet :
**07.10.92 Bulletin 92/41**

(84) Etats contractants désignés :
**AT BE CH DE DK ES FR GB GR IT LI LU NL SE**

(56) Documents cités :
**EP-A- 0 157 703**
**DE-A- 2 344 805**
**US-A- 4 015 996**

(73) Titulaire : **RHONE-POULENC CHIMIE**
**25, quai Paul Doumer**
**F-92408 Courbevoie Cédex (FR)**

(72) Inventeur : **Persello, Jacques**
**310 Chemin Calice**
**F-01120 La Boisse (FR)**

(74) Mandataire : **Dubruc, Philippe et al**
**RHONE-POULENC CHIMIE Service Brevets**
**Chimie 25, quai Paul-Doumer**
**F-92408 Courbevoie Cédex (FR)**

## Description

La présente invention concerne une silice utilisable notamment dans les compositions dentifrices, son procédé de préparation ainsi que des compositions dentifrices comprenant cette silice.

On sait que la silice est couramment utilisée dans la préparation de compositions dentifrices. Elle peut y jouer d'ailleurs plusieurs rôles.

Elle agit tout d'abord comme agent abrasif en aidant par son action mécanique à l'élimination de la plaque dentaire.

Elle peut aussi jouer le rôle d'agent épaississant pour conférer des propriétés rhéologiques déterminées au dentifrice ainsi que d'agent optique pour lui donner la coloration souhaitée.

Par ailleurs, on sait que les dentifrices contiennent généralement une source de fluorure, le plus souvent le fluorure de sodium ou le monofluorophosphate utilisé comme agent anti-carie ; un agent liant par exemple, un colloïde d'algues comme la carraghénine, la gomme guar ou la gomme xanthane ; un agent humectant qui peut être un polyalcool, par exemple, la glycérine, le sorbitol, le xylitol et le propylèneglycol. On trouve également des constituants facultatifs, par exemple, des agents tensio-actifs, des agents pour diminuer la plaque dentaire ou le dépôt du tartre, des agents correcteurs du goût ; des agents colorants et pigments etc...

Dans les compositions dentifrices peuvent intervenir un certain nombre de cations métalliques. A titre d'exemples, on peut citer les cations alcalino-terreux, notamment le calcium, le strontium, le baryum, les cations du groupe 3a, aluminium, indium, du groupe 4a, germanium, étain, plomb et du groupe 8 : manganèse, fer, nickel, zinc, titane, zirconium, palladium, etc...

Lesdits cations peuvent être sous forme de sels minéraux par exemple, chlorure, fluorure, nitrate, phosphate, sulfate ou sous forme de sels organiques acétate, citrate, etc...

Comme exemples plus spécifiques, on peut citer le citrate de zinc, le sulfate de zinc, le chlorure de strontium, le fluorure d'étain sous forme de sel simple ($SnF_2$) ou de sel double ($SnF_2$, KF), le chlorofluorure stanneux SnClF, le fluorure de zinc ($ZnF_2$).

La présence des agents contenant lesdits cations métalliques pose le problème de leur compatibilité avec la silice. En effet, à cause notamment de ses capacités adsorbantes, celle-ci peut avoir tendance à réagir avec ces agents de telle sorte qu'ils ne soient plus disponibles pour exercer la fonction qui leur est impartie.

Dans la demande de brevet française n° 87/15276, on a décrit des silices qui ont la propriété d'être compatibles avec le zinc. Toutefois, les silices décrites ne présentent pas une compatibilité suffisante avec d'autres cations métalliques tels que l'étain, le strontium, etc ...

L'objet de la présente invention est donc de fournir de nouvelles silices compatibles avec le zinc et les autres cations métalliques tels que précités.

Un autre objet de l'invention est de fournir une silice qui soit également compatible avec l'anion fluorure. En effet, l'amélioration de la compatibilité avec les cations diminue la compatibilité avec l'anion fluorure. Par conséquent, il est important que la silice proposée reste compatible avec l'anion fluorure qui se retrouve dans toutes les compositions dentifrices.

Enfin, un autre objet de l'invention est le procédé de préparation de telles silices compatibles.

Or à cet effet, la Demanderesse s'est aperçu que les propriétés de compatibilité recherchées dépendaient essentiellement de la chimie de surface de la silice utilisé. La Demanderesse a ainsi établi un certain nombre de conditions sur la surface des silices pour que celles-ci soient compatibles.

Dans ce but, la silice de l'invention utilisable notamment dans les compositions dentifrices est caractérisée en ce qu'elle présente une chimie de surface telle que le nombre d'OH⁻ exprimé en OH⁻/nm² est inférieur ou égal à 10, que son point de charge nulle (PZC) soit compris entre 3 et 6,5 et qu'elle conduit à une suspension aqueuse dont le pH varie en fonction de sa conductivité électrique selon l'équation suivante (I):

$$- pH = b - a \log (D) \quad (I)$$

dans l'équation (I):

. a est une constante inférieure ou égale à 0,6,

. b est une constante inférieure ou égale à 8,5,

. (D) représente la conductivité électrique de la suspension aqueuse de silice exprimée en microsiemens.cm⁻¹.

Une autre caractéristique de la silice de l'invention est qu'elle présente une compatibilité avec au moins un cation métallique bivalent et plus choisi dans le groupe 2a, 3a, 3a, 4a et 8 de la classification périodique d'au moins 30 %, plus particulièrement d'au moins 50 % et, préférentiellement, d'au moins 80 %.

La présente invention a également pour objet l'un des procédés de préparation de la silice de l'invention qui est caractérisé par le fait qu'il consiste à faire réagir un silicate avec un acide conduisant ainsi à une suspension ou un gel de silice, à effectuer un premier mûrissement à un pH supérieur ou égal à 6 et inférieur ou égal à 8,5, puis un deuxième mûrissement à un pH inférieur ou égal à 5,0 ; à séparer la silice ; à la soumettre à un lavage à l'eau chaude jusqu'à ce qu'il conduise à une suspension aqueuse dont le pH mesuré sur une suspension à 20 % de $SiO_2$ réponde à l'équation suivante :

$$- pH = d - c \log (D) \quad (II)$$

dans l'équation (II) :

. c est une constante inférieure ou égale à 1,0,

. d est une constante inférieure ou égale à 8,5,

. (D) représente la conductivité électrique de la suspension aqueuse de silice exprimée en microsiemens.$cm^{-1}$ ;

enfin à la sécher.

Enfin, l'invention concerne des compositions dentifrices caractérisées en ce qu'elles contiennent des silices telles que décrites ci-dessus ou préparées selon le procédé qui vient d'être mentionné plus haut.

D'autres caractéristiques et avantages de l'invention seront mieux compris à la lecture de la description et des exemples concrets qui vont suivre.

Comme cela a été indiqué, en introduction, les caractéristiques essentielles des silices de l'invention résident dans leur chimie de surface. Plus précisément, un des aspects à prendre en compte dans cette chimie de surface est l'acidité. A ce sujet, une des caractéristiques des silices de l'invention est le nombre des sites acides de surface.

Ce nombre se mesure en nombre de groupements $OH^-$ ou silanols par $nm^2$. En pratique, la mesure se fait de la manière suivante.

La détermination de ce nombre se fait de la manière suivante : le nombre de sites $OH^-$ de surface est assimilé à la quantité d'eau libérée par la silice entre 190°C et 900°C.

Les échantillons de silice sont préalablement séchés à 105°C pendant 2 heures.

Une masse $P_o$ de silice est placée dans une thermobalance et portée à 190°C pendant 2 heures : soit $P_{190}$, la masse obtenue.

La silice est portée ensuite à 900°C pendant 2 heures, soit $P_{900}$ la nouvelle masse obtenue.

Le nombre de sites $OH^-$ est calculé par l'équation suivante :

$$N_{OH^-} = \frac{66922,2}{A} \times \frac{P_{190} - P_{900}}{P_{190}}$$

dans laquelle :

— $N_{OH^-}$ est le nombre de sites $OH^-$ par $nm^2$ de surface

— A est la surface spécifique du solide mesurée par BET et exprimée en $m^2/g$.

Dans le cas présent, les silices de l'invention présentent avantageusement un nombre d'$OH^-$/$nm^2$ inférieur ou égal à 10, plus particulièrement compris entre 4 et 10.

La nature des sites $OH^-$ des silices de l'invention qui constitue aussi une caractérisation de leur chimie de surface peut aussi être appréciée par le point de charge nulle.

Ce point de charge nulle (PZC) est défini par le pH d'une suspension de silice pour lequel la charge électrique de la surface du solide est nulle et cela quelle que soit la force ionique du milieu. Ce PZC mesure le pH réel de la surface, dans la mesure où celle-ci est libre de toutes impuretés de type ionique.

La charge électrique est déterminée par potentiomètrie. Le principe de la méthode est basé sur le bilan global des protons adsorbés ou désorbés sur la surface de la silice à un pH donné.

A partir des équations décrivant le bilan global de l'opération, il est facile de montrer que la charge électrique c de la surface, prise par rapport à une référence correspondant à une charge de surface nulle, est donnée par l'équation :

$$c = \frac{F}{A.M} (H^+) - (OH^-)$$

dans laquelle :

— A représente la surface spécifique du solide en $m^2/g$,

— M est la quantité de solide dans la suspension en g,

— F est le Faraday,

— $(H^+)$ ou $(OH^-)$ représente la variation par unité de surface de l'excès d'ions $H^+$ ou $OH^-$ respectivement sur le solide.

Le protocole expérimental de détermination du PZC est le suivant :

On utilise la méthode décrite par Berube et de Bruyn (J. Colloid Interface Sc 1968, 27, 305).

La silice est lavée au préalable dans de l'eau désionisée de haute résistivité (10 Méga.Ohm.cm), séchée puis dégazée.

Pratiquement, on prépare une série de solutions à $pH_o = 8,5$ par ajout de KOH ou $HNO_3$ et contenant un électrolyte indifférent ($KNO_3$) à une concentration variable entre $10^{-5}$ et $10^{-1}$ Mole/l.

A ces solutions, on ajoute une masse donnée de silice et on laisse le pH des suspensions obtenues se stabiliser sous agitation, à 25°C et sous azote pendant 24 heures ; soit pH'o sa valeur.

Des solutions étalons sont constituées par le surnageant obtenu par centrifugation pendant 30 mn à 1000 t/mn d'une partie de ces mêmes suspensions ; soit pH'o, le pH de ces surnageants.

On ramène ensuite le pH d'un volume connu de ses suspensions et des solutions étalons correspondantes, à pHo en rajoutant la quantité nécessaire de KOH et on laisse les suspensions et les solutions étalons se stabiliser pendant 4 heures.

Soit $V_{OH^-}$ . $N_{OH^-}$ le nombre d'équivalents de base ajoutée pour asser de pH'o à pHo d'un volume (V) connu de suspension ou de solution étalon.

Le dosage potentiomètrique des suspensions et des solutions étalons est effectué à partir de pHo par addition d'acide nitrique jusqu'à pHf = 2,0.

Préférentiellement, on procède par addition d'incrément d'acide correspondant à une variation de pH de 0,2 unité de pH. Après chaque addition, le pH est stabilisé pendant 1 mn.

Soit $V_{H^+}$ . $N_{H^+}$ le nombre d'équivalents d'acide pour parvenir à pHf.

A partir de pHo, on trace le terme $(V_{H^+} . N_{H^+} - V_{OH^-} . N_{OH^-})$ en fonction des pH incrémentés pour toutes les suspensions (3 forces ioniques au moins) et pour toutes les solutions étalons correspondantes.

Pour chaque valeur de pH (pas de 0,2 unité), on fait ensuite la différence entre la consommation de $H^+$ ou $OH^-$ pour la suspension et pour la solution étalon correspondante. On renouvelle cette opération pour toutes les forces ioniques.

Ceci donne le terme $(H^+)$ - $(OH^-)$ correspondant à la consommation en protons de la surface. La charge de surface est calculée par l'équation ci-dessus.

On trace ensuite les courbes charge de surface en fonction du pH pour toutes les forces ioniques considérées. Le PZC est défini par l'intersection des courbes.

On ajuste la concentration en silice en fonction de la surface spécifique de celle-ci.

Par exemple, on utilise des suspensions à 2 % pour les silices de 50 m²/g à 3 forces ioniques (0,1 ; 0,01 et 0,001 mole/l).

Le dosage est effectué sur 100 ml de suspension en utilisant de l'hydroxyde de potassium à 0,1 M.

Pour les silices de l'invention ce PZC doit être compris entre 3 et 6,5.

Par ailleurs, pour améliorer la compatibilité des silices de l'invention vis-à-vis d'autres éléments notamment vis-à-vis du fluor, il est intéressant que la teneur en cations bivalents et plus contenues dans la silice soit au plus égale à 1000 ppm. Notamment, il est souhaitable que la teneur en aluminium des silices de l'invention soit au plus de 500 ppm.

D'autre part, la teneur en fer des silices de l'invention peut être avantageusement d'au plus 200 ppm.

Par ailleurs, d'une manière préférentielle, la teneur en calcium peut être d'au plus 500 ppm et plus particulièrement d'au plus 300 ppm.

Les silices de l'invention présentent aussi de préférence une teneur en carbone d'au plus 50 ppm et plus particulièrement d'au plus 10 ppm.

Enfin, le pH des silices selon l'invention mesuré selon la norme NFT 45-007 est généralement au plus de 7. Il est plus particulièrement compris entre 6 et 7.

Les caractéristiques ci-dessus permettent d'avoir une silice qui soit compatible avec les cations métalliques bivalents et plus, en particuler, zinc, strontium, étain. Cette compatibilité mesurée selon le test donné ci-dessous, est d'au moins 30 %, plus particulièrement d'au moins 50 % et préférentiellement d'au moins 80 %.

De plus, les silices de l'invention peuvent présenter une bonne compatibilité avec l'anion, fluorure d'au moins environ 80 % et, de préférence, d'au moins 90 %.

Outre les caractéristiques de chimie de surface qui viennent d'être décrites ci-dessous et qui conditionnent les compatibilités, les silices de l'invention présentent arussi des caractéristiques physique) qui les rendent parfaitement adaptées à leur application en dentifrice. Ces caractéristiques de type structurel vont être décrites ci-dessous.

Généralement, la surface BET des silices de l'invention est comprise entre 40 et 600 m²/g. Leur surface CTAB varie habituellement entre 40 et 400 m²/g.

La surface BET est déterminée selon la méthode de BRUNAUER-EMMET-TELLER décrite dans the Journal of the American Chemical Society vol. 60, page 309, February 1938 et selon la norme NF X11-622(3.3).

La surface CTAB est la surface externe déterminée selon la norme ASTM D3765 mais en pratiquant l'adsorption de bromure d'hexadécyltriméthyl ammonium (CTAB) à pH 9 et en prenant comme aire projetée de la molécule de CTAB 35 Å².

Les silices de l'invention peuvent bien entendu correspondre aux trois types habituellement distingués dans le domaine du dentifrice.

Ainsi, les silices de l'invention peuvent être du type abrasif. Elles présentent alors une surface BET comprise entre 40 et 300 m²/g. Dans ce cas, la surface CTAB est comprise entre 40 et 100 m²/g.

Les silices de l'invention peuvent aussi être du type épaississant. Elles ont alors une surface BET comprise entre 120 et 450 m²/g, plus particulièrement 120 et 200 m²/g. Elles pourront présenter alors une surface CTAB entre 120 et 400 m²/g, plus particulièrement entre 120 et 200 m²/g.

Enfin, selon un troisième type, les silices de l'invention peuvent être bifonctionnelles. Elles possèdent ici une surface BET comprise entre 80 et 200 m²/g. La surface CTAB est alors comprise entre 80 et 200 m²/g.

Les silices de l'invention peuvent aussi présenter une prise d'huile comprise entre 80 et 500 cm³/100 g déterminée selon la norme NFT 30-022 (mars 53) en mettant en oeuvre le phtalate de dibutyle.

Plus précisément, cette prise d'huile sera comprise entre 100 et 140 cm³/100 g pour les silices abrasives, 200 et 400 pour les silices épaississantes et 100 et 300 pour les bifonctionnnelles.

Par ailleurs, toujours en vue de l'application en dentifrice, les silices ont préférentiellement une taille de particules comprise entre 1 et 10 µm.

La densité apparente variera généralement entre 0,01 et 0,3.

Selon un mode de réalisation particulier de l'invention, les silices sont des silices de précipitation.

Enfin, les silices de l'invention présentent un indice de réfraction généralement compris entre 1,440 et 1,465.

Le procédé de préparation des silices de l'invention va maintenant être décrit plus particulièrement.

Comme indiqué plus haut, ce procédé est du type comprenant la réaction d'un silicate avec un acide ce qui donne lieu à la formation d'une suspension ou d'un gel de silice.

Il est à noter que l'on peut utiliser tout mode opératoire connu pour arriver à cette suspension ou ce gel, (addition d'acide sur un pied de cuve de silicate, addition simultanée totale ou partielle d'acide et de silicate sur un pied de cuve d'eau ou de solution de silicate etc...) le choix se faisant essentiellement en fonction des caractéristiques physiques de la silice que l'on désire obtenir.

Un mode de réalisation préférentiel de l'invention consiste à préparer la suspension ou le gel de silice en ajoutant simultanément le silicate et l'acide sur un pied de cuve qui peut être de l'eau, une dispersion colloïdale de silice contenant de 0 à 150 g/l de silice exprimée en $SiO_2$, un silicate ou un sel minéral ou organique, de préférence de métal alcalin tel que, par exemple, le sulfate de sodium, l'acétate de sodium.

L'addition des deux réactifs se fait de manière simultanée de telle sorte que le pH soit maintenu constant entre 4 et 10, de préférence entre 8,5 et 9,5. La température est avantageusement comprise entre 60°C et 95°C.

Un mode de préparation de la dispersion colloïdale de silice ayant de préférence une concentration entre 20 et 150 g/l consiste à chauffer une solution aqueuse de silicate par exemple entre 60°C et 95°C et à ajouter l'acide dans ladite solution aqueuse jusqu'à obtention d'un pH compris entre 8,0 et 10,0 de préférence aux environs de 9,5.

La concentration de la solution aqueuse de silicate exprimée en $SiO_2$ est de préférence comprise entre 20 et 150 g/l. On peut faire appel à un acide dilué ou concentré : sa normalité pouvant varier entre 0,5 N et 36 N, de préférence entre 1 et 2 N.

Dans ce qui précède, on entend par silicate avantageusement un silicate de métal alcalin et, de préférence, un silicate de sodium de rapport pondéral $SiO_2/Na_2O$ compris entre 2 et 4, de préférence égal à 3,5. Pour ce qui est de l'acide, il peut être gazeux, comme le gaz carbonique ou liquide, de préférence, l'acide sulfurique.

Dans une autre étape du procédé de l'invention, on soumet la suspension ou le gel à une opération de double mûrissement.

On effectue un premier mûrissement à un pH d'au plus 8,5 et compris entre 6 et 8,5, par exemple, de préférence à 8,0. Le mûrissement se fait de préférence à chaud, par exemple à une température comprise entre 60°C et 100°C, de préférence 95°C et sur une durée qui peut varier entre 10 minutes et 2 heures.

Une autre variante d'exécution de l'invention consiste à préparer une suspension ou un gel de silice en ajoutant progressivement l'acide dans un pied de cuve contenant le silicate jusqu'à obtention du pH de mûrissement désiré. Cette opération est conduite à une température comprise, de préférence, entre 60°C et 95°C. On effectue ensuite le mûrissement de la suspension du gel de silice dans les conditions décrites précédemment.

On réalise ensuite un deuxième mûrissement à un pH inférieur à 5, de préférence, compris entre 3 et 5 et, encore plus préférentiellement, entre 3,5 et 4,0.

Les conditions de température et de durée sont celles du premier mûrissement.

A cet effet, on amène le pH au pH de mûrissement souhaité par ajout d'acide.

On peut faire appel, par exemple, à un acide minéral tel que l'acide nitrique, chlorhydrique, sulfurique, phosphorique ou bien à l'acide carbonique formé par barbotage de gaz carbonique.

On procède alors à la séparation de la silice du milieu réactionnel selon tout moyen connu, filtre sous vide ou filtre presse par exemple.

On recueille ainsi un gâteau de silice.

EP 0 396 460 B1

Dans l'étape suivante du procédé de l'invention, on effectue le lavage du gâteau de silice obtenue. Le lavage est réalisé dans des conditions telles que le pH de la suspension ou du milieu avant séchage réponde à l'équation suivante :

$$- pH = d - e \log (D) \quad (II)$$

dans l'équation (II) :

. e est une constante inférieure ou égale à 1,0,

. d est une constante inférieure ou égale à 8,5

. (D) représente la conductivité électrique de la suspension aqueuse de silice exprimée en microsiemens.cm$^{-1}$.

On réalise le lavage à l'eau ayant de préférence une température comprise entre 40°C et 80°C. On peut effectuer selon le cas, un ou plusieurs lavages, généralement deux, à l'eau, de préférence désionisée et/ou à l'aide d'une solution acide ayant un pH compris entre 2 et 7.

Cette solution acide peut être par exemple une solution d'un acide minéral tel que l'acide nitrique.

Toutefois selon un mode de réalisation particulier de l'invention, cette solution acide peut aussi être une solution d'un acide organique notamment d'un acide organique complexant. Cet acide pourra être choisi dans le groupe des acides carboxyliques, dicarboxyliques, hydroxycarboxyliques et amino-carboxyliques.

On peut citer comme exemple de tels acides l'acide acétique et pour les acides complexants, l'acide tartrique, l'acide maléique, l'acide glycérique, l'acide gluconique, l'acide citrique.

Il peut être avantageux, surtout dans le cas de l'emploi d'une solution d'un acide minéral, de procéder à un ultime lavage par de l'eau désionisée.

D'un point de vue pratique, les opérations de lavage peuvent se faire par passage de la solution de lavage sur le gâteau ou par introduction de celle-ci dans la suspension obtenue, après délitage du gâteau.

En effet le gâteau de filtration est soumis avant l'opération de séchage à un délitage qui peut être effectué par tout moyen connu, par exemple, à l'aide d'un agitateur tournant à grande vitesse.

Le gâteau de silice, avant ou après lavage, est donc délité puis séché selon tout moyen connu. Le séchage peut se faire par exemple, dans un four tunnel ou à moufle ou par atomisation dans un courant d'air chaud dont la température d'entrée peut varier entre 200°C et 500°C environ et la température de sortie entre 80°C et 100°C environ : le temps de séjour étant compris entre 10 secondes et 5 minutes.

Le produit séché peut être broyé si nécessaire pour obtenir la granulométrie désirée. L'opération est conduite dans un appareillage classique : broyeur à couteaux ou broyeur à jet d'air.

L'invention concerne aussi des compositions dentifrices contenant les silices du type décrit ci-dessus ou obtenues par le procédé qui vient d'être étudié.

La quantité de silice selon l'invention utilisée dans les compositions dentifrices peut varier dans de larges limites : elle est habituellement comprise entre 5 et 35 %.

Les silices de l'invention s'appliquent particulièrement bien aux compositions dentifrices contenant au moins un élément choisi dans le groupe comprenant les fluorures, les phosphates, les cations métalliques.

En ce qui concerne les composés fluorés, leur quantité correspond de préférence à une concentration en fluor dans la composition comprise entre 0,01 et 1 % en poids et plus particulièrement 0,1 % à 0,5 %. Les composés fluorés sont en particulier les sels de l'acide monofluorophosphorique et particulièrement ceux de sodium, potassium, lithium, calcium, aluminium et ammonium, le mono et le difluorophosphate ainsi que des fluorures variés contenant le fluor sous forme d'ion lié particulièrement les fluorures alcalins comme ceux de sodium, lithium, potassium, le fluorure d'ammonium, le fluorure stanneux, le fluorure de manganèse, le fluorure de zirconium, le fluorure d'aluminium ainsi que des produits d'addition de ces fluorures entre eux ou avec d'autres fluorures, tels que les fluorures de potassium ou de sodium ou de manganèse.

D'autres fluorures sont également utilisables pour la présente invention comme, par exemple, le fluorure de zinc, le fluorure de germanium, le fluorure de palladium, le fluorure de titane, les fluozirconates alcalins par exemple de sodium ou de potassium, le fluozirconate stanneux, le fluoborate ou les fluosulfates de sodium, de potassium.

Les composés fluorés organiques peuvent également être utilisés, de préférence ceux connus comme les produits d'addition d'amines ou d'amino-acides à longue chaîne avec le fluorure d'hydrogène, tels que le l'hydrofluorure de cétylamine, le dihydrofluorure de bis-(hydroxyéthyl)aminopropyl N-hydroxyéthyl octadécylamine, le fluorure d'octadécylamine et le dihydrofluorure de N, N', N'tri-(polyoxyéthylène) N-hexadécylpropylènodiamine.

En ce qui concerne les composants apportant les cations métalliques bivalents et plus, parmi ceux précités dans la description les plus couramment rencontrés sont le citrate de zinc, le sulfate de zinc, le chlorure de strontium, le fluorure d'étain.

Pour les éléments utilisables comme agents anti-plaques du type polyphosphates ou polyphosphonates, guanidines, bis-biguanides on peut mentionner ceux indiqués dans les brevets US 3.934.002

Les compositions dentifrices peuvent contenir en outre un liant.

6

Les principaux liants utilisés sont notamment choisis parmi :
- les dérivés cellulosiques : méthylcellulose, hydroxyéthylcellulose, carboxyméthylcellulose sodique,
- les mucilages : carraghénates, alginates, agar-agar et géloses,
- les gommes : gommes arabique et adragante, gomme xanthane, gomme karaya,
- les polymères carboxyvinyliques et acryliques,
- les résines de polyoxyéthylène.

Outre les silices de l'invention, les compositions dentifrices peuvent contenir aussi un ou plusieurs autres agents abrasifs polissants choisis notamment parmi :
- le carbonate de calcium précipité,
- le carbonate de magnésium,
- les phosphates de calcium, di- et tricalciques,
- le métaphosphate de sodium insoluble,
- le pyrophosphate de calcium,
- l'oxyde de titane (agent de blanchiment),
- les silicates,
- les alumines et silico-aluminates,
- les oxydes de zinc et d'étain,
- le talc,
- le kaolin.

Les compositions dentifrices peuvent aussi comprendre des tensio-actifs, des humectants, des agents aromatisants, édulcorants et des colorants et des conservateurs.

Les principaux tensio-actifs utilisés sont notamment choisis parmi :
- le laurylsulfate de sodium,
- le lauryléthersulfate et le laurylsulfoacétate de sodium,
- le dioctylsulfosuccinate de sodium,
- le laurylsarcosinate de sodium,
- le ricinoléate de sodium,
- les monoglycérides sulfatés.

Les principaux agents humectants utilisés sont notamment choisis parmi les polyalcools comme :
- le glycérol,
- le sorbitol, généralement en solution à 70 % dans l'eau,
- le propylène glycol.

Les principaux agents aromatisants (parfum) sont notamment choisis parmi : les essences d'anis, de badiane, de menthe, de baie de genièvre, de cannelle, de girofle et de rose.

Les principaux agents édulcorants sont notamment choisis parmi les imides orthosulfobenzoïques et le cyclamates.

Les principaux colorants utilisés sont notamment choisis selon la couleur désirée parmi :
- coloration rouge et rose : amaranthe, azorubine, cachou, coccine nouvelle (PONCEAU 4 R), cochenille, érythrosine,
- coloration verte : chlorophylle et chlorophylline,
- coloration jaune : jaune soleil (Orange S) et jaune de quinoléine.

Les principaux conservateurs les plus utilisés sont : les parahydroxybenzoates, le formol et les produits qui en dégagent, l'héxétidine, les ammoniums quaternaires, l'hexachlorophène, le bromophène et l'hexamédine.

Enfin, les compositions dentifrices contiennent des agents thérapeutiques dont les principaux sont notamment choisis parmi :
- les antiseptiques et les antibiotiques,
- les enzymes,
- les oligo-éléments et les composés fluorés qui ont été décrits ci-dessus.

Des exemples concrets mais non limitatifs de l'invention vont maintenant être donnés.

Auparavant, le protocole de mesure du pH en fonction de la conductivité et de la concentration ainsi que les tests pour la mesure de la compatibilité de la silice avec différents éléments vont être décrits.

## Protocole de mesure du pH en fonction de la concentration en silice et de la conductivité

Des suspensions de silice de concentration croissante variant de 0 à 25 % en poids sont constituées en dispersant une masse m de silice préalablement séchées à 120°C pendant 2 heures dans une masse 100-m d'eau désionisée et dégazée (Qualité Millipore). Les suspensions sont agitées pendant 24 heures à 25°C.

7

Le pH des suspensions et des solutions obtenues après centrifugation d'une partie de la suspension à 8000 tours/mn pendant 40 mn et filtration sur filtre Millipore 0,22 µm, sont mesurés à 25°C sous atmosphère d'azote avec un système de mesure de type Titroprocessor Metrohm 672.

De la même façon, on mesure la conductivité des suspensions et des solutions obtenues comme précédemment à 25°C avec un conductivimètre Radiometer (CDM83) équipé d'une cellule de type CDC304 de constante de cellule égale à 1 cm-1. La conductivité est exprimée en µSiemens/cm.

L'effet de suspension (SE) est défini par la différence de pH entre le pH d'une suspension à 20 % en silice et le pH de sa solution surnageante séparée par centrifugation.

Mesure de la compatibilité avec le zinc

4 g de silice sont dispersés dans 100 ml de solution à 0,06 % de $ZnSO_4$, $7H_2O$. On obtient une suspension dont le pH est stabilisé à 7 pendant 15 minutes par ajout de NaOH ou $H_2SO_4$. La suspension est agitée ensuite pendant 24 heures à 37°C puis est centrifugée à 20000 t/mn pendant 30 mn.

Le surnageant filtré sur filtre Millipore 0,2 µm, constitue la solution de l'essai.

Une solution de référence est constituée en suivant le même protocole mais en absence de silice.

La concentration en zinc libre des deux solutions est déterminée par absorption atomique (214 nm).

La compatibilité est déterminée par la relation ci-dessous :

$$\% \text{ compatibilité } = \frac{\text{Concentration en Zn de l'essai (ppm)}}{\text{Concentration en Zn de la référence (ppm)}} \times 100$$

Par la suite, % de compatibilité zinc est désigné par Zn.

Mesure de compatibilité avec le fluorure d'étain $SnF_2$

1) Une solution aqueuse (1) contenant 0,40 % de $SnF_2$ et 20 % de glycérine est constituée en dissolvant 0,40 g de $SnF_2$ et 20 g de glycérine dans 79,60 g d'eau bi-distillée.

2) On disperse 4 g de silice dans 16 g de solution obtenue en 1). On ajuste le pH des suspensions à 5 par ajout de NaOH 0,1 N.

La suspension ainsi obtenue est agitée pendant 4 semaines à 37°C.

3) La suspension est ensuite centrifugée à 8000 tours/mn pendant 30 mn et le surnageant obtenu (3) est filtré sur filtre Millipore 0,22 µm.

4) La concentration en étain libre est déterminée par absorption atomique dans la solution obtenue en 1) et dans le surnageant obtenu en 3).

5) La compatibilité est déterminée par la relation ci-dessous :

$$\% \text{ compatibilité } = \frac{\text{concentration en Sn dans le surnageant (3)}}{\text{concentration en Sn dans la solution (1)}} \times 100$$

Par la suite, % Compatibilité étain est désigné par Sn.

Mesure de compatibilité avec le chlorure de strontium $SrCl_2,6H_2O$

1) Une solution aqueuse (1) contenant 1 % de $SrCl_2$, $6H_2O$ est constituée en dissolvant 1 g de $SrCl_2$, $6H_2O$ dans 99 g d'eau bi-distillée. On ajuste le pH des suspensions à 7,0 par ajout de NaOH 0,1 N.

2) On disperse 4 g de silice dans 16 g de solution obtenue en 1).

La suspension ainsi obtenue est agitée pendant 4 semaines à 37°C.

3) La suspension est ensuite centrifugée à 8000 tours/mn pendant 30 mn et le surnageant obtenu (3) est filtré sur filtre Millipore 0,22 µm.

4) La concentration en strontium libre est déterminée par absorption atomique dans la solution obtenue en 1) et dans le surnageant obtenu en 3).

5) La compatibilité est déterminée par la relation ci-dessous :

$$\% \text{ compatibilité } = \frac{\text{concentration en Sr dans le surnageant (3)}}{\text{concentration en Sr dans la solution (1)}} \times 100$$

Par la suite, % Compatibilité strontium est désigné par Sr.

Mesure de la compatibilité avec les fluorures

4 g de silice sont dispersés dans 16 g de solution à 0,3 % de fluorure de sodium (NaF). La suspension est agitée pendant 24 heures à 37°C. Après centrifugation de la suspension à 20000 t/mn pendant 30 mn, le surnageant est filtré sur filtre Millipore 0,2 µm. La solution ainsi obtenue, constitue la solution de l'essai.

Une solution de référence est constituée en utilisant le même protocole mais en absence de silice.

La compatibilité avec les fluorures est déterminée par le % de fluorure libre mesuré par électrode sélective à fluorure (Orion). Elle est déterminée par la relation ci-dessous.

$$\% \text{ compatibilité} = \frac{\text{Concentration en F de l'essai (ppm)}}{\text{Concentration en F de la référence (ppm)}} \times 100$$

Mesure de la compatibilité avec les pyrophosphates de sodium et de potassium

4 g de silice sont dispersés dans 16 g de suspension à 1,5 % de pyrophosphate de sodium ou de potassium. La suspension est agitée pendant 24 heures à 37°C puis est centrifugée à 20000 t/mn pendant 30 mn.

Le surnageant est filtré sur filtre Millipore 0,2 μm. 0,2 g de solution diluée dans 100 ml d'eau dans une fiole jaugée, constitue la solution de l'essai.

Une solution de référence est constituée en suivant le même protocole mais en absence de silice.

La concentration en ion pyrophosphate ($P_2O_7^{--}$) libre des deux solutions est déterminée par chromatographie ionique (système DIONEX 2000i) équipé d'un intégrateur.

La compatibilité est déterminée par le rapport des aires des pics obtenus sur les chromatogrammes et correspondant au temps de rétention du pyrophosphate, de l'essai et de la référence.

$$\% \text{ compatibilité} = \frac{\text{Aire du pic de l'essai}}{\text{Aire du pic de la référence}} \times 100$$

## EXEMPLE 1

Dans un réacteur équipé d'un système de régulation de température et de pH et d'un système d'agitation à hélice (Mixel), on introduit 8,32 l de silicate de sodium de concentration en silice de 130 g/l et de rapport molaire $SiO_2/Na_2O = 3,5$ et 8,33 l d'eau permutée de conductivité 1 μS/cm.

Après la mise en marche de l'agitation (350 t/mn), le pied de cuve ainsi constitué est chauffé à 90°C.

Lorsque la température est atteinte, on procède à l'addition d'acide sulfurique de concentration 80 g/l avec un débit constant de 0,40 l/mn pour amener le pH à 9,5.

On procède par la suite à l'addition simultanée de 45,25 l de silicate de sodium de concentration en silice de 130 g/l, de rapport molaire $SiO_2/Na_2O = 3,5$ et de débit égal à 0,754 l/mn et de 29,64 l d'acide sulfurique à 80 g/l. Le débit d'acide sulfurique et ajusté de manière à maintenir le pH du milieu réactionnel à une valeur constante de 9,5.

Après 60 mn d'addition, on arrête l'addition de silicate de sodium et on continue l'ajout d'acide sulfurique avec un débit de 0,494 l/mn jusqu'à stabilisation du pH du mélange réactionnel à 8,0. Pendant cette phase, la température du milieu est portée à 95°C. On réalise, par la suite, un mûrissement de 30 mn à ce pH et à 95°C. Pendant le mûrissement, le pH est maintenu à 8 par ajout d'acide.

En fin de mûrissement, le pH est amené à 3,5 par ajout d'acide sulfurique. Ce pH est maintenu à 3,5 pendant 30 mn.

Après arrêt du chauffage, le mélange est filtré et le gâteau obtenu est lavé avec 20 l d'eau désionisée et chauffée à 80°C. Le gâteau obtenu après lavage est dispersé en présence d'eau désionisée pour former une suspension de concentration en silice égale à 10 %.

On procède à une deuxième filtration suivie d'un lavage à l'eau de manière à ajuster la conductivité à 500 μS/cm et d'un lavage avec de l'eau de pH ajusté à 5 par l'acide citrique de manière à ajuster le pH à une valeur inférieure à 6. On procède à un dernier lavage à l'eau désionisée.

On vérifie que le pH de la suspension aqueuse du gâteau délité ayant une teneur en $SiO_2$ de 20 % vérifie la relation suivante :

$$pH \leq 8,20 - 0,91 \log (D)$$

La silice est séchée par atomisation. On procède enfin à un broyage de la silice obtenue sur un broyeur à couteaux pour obtenir une poudre dont le diamètre moyen des agglomérats mesuré au COUNTER-COULTER est de 8 μm.

Les caractérisations physico-chimiques de la silice ainsi obtenue sont regroupées dans le tableau ci-dessous :

| | |
|---|---|
| Surface BET m²/g | 65 |
| Surface CTAB m²/g | 60 |
| Prise DOP ml/100 g silice | 125 |
| Volume poreux Hg cm³/g | 2,1 |
| pH (5 % d'eau) | 6,2 |
| Indice de réfraction | 1,450 |

| | |
|---|---|
| Translucidité % | 90 |
| $SO_4^=$ ppm | 100 |
| $Na^+$ ppm | 60 |
| $Al^{3+}$ ppm | 150 |
| $Fe^{3+}$ ppm | 100 |
| $Ca^{2+}$ ppm | 10 |
| $Cl^-$ ppm | 20 |
| C ppm | 20 |

On récapitule dans le Tableau I les caractéristiques de chimie de surface de la silice de l'invention et l'on donne dans le Tableau II les résultats de la compatibilité avec les cations métalliques : zinc, étain, strontium et avec les composants classiques des formulations de dentifrice : fluorure, pyrophosphate.

## EXEMPLE 2

Dans un réacteur équipé d'un système de régulation de température et de pH et d'un système d'agitation à hélice (Mixel), on introduit 5,30 l de silicate de sodium de concentration en silice de 135 g/l et de rapport molaire $SiO_2/Na_2O$ = 3,5 et 15,00 l d'eau permutée de conductivité 1 µS/cm.

Après la mise en marche de l'agitation (350 t/mn), le pied de cuve ainsi constitué est chauffé à 90°C.

Lorsque la température est atteinte, on procède à l'addition d'acide sulfurique de concentration 80 g/l avec un débit constant de 0,38 l/mn pour amener le pH à 9,5.

On procède par la suite à l'addition simultanée de 44,70 l de silicate de sodium de concentration en silice de 135 g/l, de rapport molaire $SiO_2/Na_2O$ = 3,5 et de débit égal à 0,745 l/mn et de 25,30 l d'acide sulfurique à 80 g/l. Le débit d'acide sulfurique est ajusté de manière à maintenir le pH du milieu réactionnel à une valeur constante de 9,5.

Après 60 mn d'addition, on arrête l'addition de silicate de sodium et on continue l'ajout d'acide sulfurique avec un débit de 0,350 l/mn jusqu'à stabilisation du pH du mélange réactionnel à 7,0. Pendant cette phase, la température du milieu est portée à 95°C. On réalise, par la suite, un mûrissement de 30 mn à ce pH et à 95°C. Pendant le mûrissement, le pH est maintenu à 7 par ajout d'acide

En fin de mûrissement, le pH est amené à 4,0 par ajout d'acide sulfurique. Ce pH est maintenu à 4,0 pendant 30 mn.

Après arrêt du chauffage, le mélange est filtré et le gâteau obtenu est lavé à l'eau désionisée jusqu'à obtention d'un filtrat de conductivité égale à 2000 µS/cm.

Le gâteau est ensuite délité en présence d'eau pour former une suspension à 20 % en silice.

On procède à un dernier lavage à l'eau désionisée de manière à ce que le pH de la suspension aqueuse du gâteau délité ayant une teneur en $SiO_2$ de 20 % vérifie la relation suivante :

$$pH \leq 8,20 - 0,91 \log (D)$$

La silice est séchée à 120°C pendant 24 heures puis broyée sur un broyeur à couteaux pour obtenir une poudre dont le diamètre moyen des agglomérats est de 8 µm.

Les caractérisations physico-chimiques de la silice ainsi obtenue sont regroupées dans le tableau ci-dessous :

| | |
|---|---|
| Surface BET $m^2/g$ | 85 |
| Surface CTAB $m^2/g$ | 80 |
| Prise DOP ml/100 g silice | 150 |
| Volume poreux Hg $cm^3/g$ | 3,20 |
| pH (5 % eau) | 6,5 |
| Indice de réfraction | 1,455 |
| Translucidité % | 70 |
| $SO_4^=$ % | 0,5 |
| $Na^+$ % | 0,05 |
| $Al^{3+}$ ppm | 250 |
| $Fe^{3+}$ ppm | 120 |
| $Ca^{2+}$ ppm | 50 |
| $Cl^-$ ppm | 20 |
| C ppm | 5 |

On récapitule dans le Tableau I, ci-après, les caractéristiques de chimie de surface des silices de l'invention décrites dans les exemples 1 et 2.

On donne également dans le Tableau I, les résultats de compatibilité des silices de l'invention avec les cations métalliques : zinc, étain, strontium et avec les composants classiques des formulations de dentifrice :

fluorure, pyrophosphate.

A titre comparatif, on consigne dans les Tableaux I et II, les caractéristiques et les différentes compatibilités de silices commerciales dont la liste ci-après définie constitue une gamme représentative des silices classiques.

S81 :    Syloblanc 81 (GRACE)
Z113 :    Zeodent 113 (HUBER)
Sid12 :    Sident 12 (DEGUSSA)
Syl15 :    Sylox 15 (GRACE)
T73 :    Tixosil 73 (RHONE-POULENC)
T83 :    Tixosil 83 (RHONE-POULENC)

## Tableau I

### Caractéristiques physico-chimiques des silices de l'invention et des silices classiques.

|  | Caractéristiques Physico-Chimiques des Silices | | | |
|---|---|---|---|---|
| silice | pH log(D) | SE | Ho | PZC |
| S81 | 7,0-0,62z | - 0,17 | ≤ 2 | 2,2 |
| Z113 | 10-1,0z | - 0,70 | ≤ 3 | 2,5 |
| Sid12 | 8,5-0,60z | - 0,20 | ≤ 3 | 2,8 |
| Syl15 | 9,2-0,74z | - 0,94 | ≤ 3 | 2,5 |
| T73 | 10-0,87z | - 0,20 | ≤ 3 | 3,0 |
| T83 | 8,6-0,60z | - 0,18 | ≤ 3 | 2,5 |
| Ex 1 | 8,0-0,50z | - 0,00 | ≥ 4 | 4,2 |
| Ex 2 | 7,4-0,30z | - 0,03 | ≥ 4 | 4,0 |

La signification des symboles utilisés dans le tableau ci-dessus est donnée ci-dessous :

– pH/log(D) représente l'équation pH = b-a log(D) où b et a sont deux constantes et D est la conductivité de la suspension de silice en $\mu$Siemens/cm.

– SE représente l'effet de suspension mesuré par la relation SE = pH suspension - pH surnageant définie par ailleurs

– Ho est la constante de Hammett

– PZC représente le pH pour lequel la charge de surface de la silice est nulle.

Tableau II

Compatibilités des silices avec les molécules actives :

| silice | % Compatibilité | | | | |
|---|---|---|---|---|---|
| | Zn | Sn | Sr | F | $P_2O_7$ |
| S81 | 0 | 25 | 20 | 90 | 80 |
| Z113 | 0 | 15 | 10 | 95 | 90 |
| Sid12 | 10 | 25 | 20 | 90 | 80 |
| Syl15 | 0 | 10 | 10 | 90 | 80 |
| T73 | 20 | 15 | 10 | 90 | 90 |
| T83 | 10 | 10 | 10 | 95 | 95 |
| Ex 1 | 80 | 60 | 90 | 95 | 95 |
| Ex 2 | 85 | 75 | 95 | 95 | 90 |

Les silices de l'invention se distinguent des silices classiques par leurs caractéristiques physico-chimiques et par leur bonne compatibilité avec le zinc, l'étain et le strontium.

**Revendications**

**Revendications pour les Etats contractants suivants : AT, BE, CH, DE, DK, FR, GB, GR, IT, LI, LU, NL, SE**

1 - Silice caractérisée par le fait qu'elle présente une chimie de surface telle que le nombre d'$OH^-$ exprimé en $OH^-/nm^2$ est inférieur ou égal à 10, que son point de charge nulle (PZC) soit compris entre 3 et 6,5 et qu'elle conduit à une suspension aqueuse dont le pH varie en fonction de sa conductivité électrique selon l'équation suivante (I) :

$$pH = b - a \log (D) \quad (I)$$

dans l'équation (I) :
. a est une constante inférieure ou égale à 0,6,
. b est une constante inférieure ou égale à 8,5,
. (D) représente la conductivité électrique de la suspension aqueuse de silice exprimée en microsiemens.$cm^{-1}$.

2 - Silice selon la revendication 1 caractérisée par le fait qu'elle présente une chimie de surface telle que le nombre de $OH^-$ exprimé en $OH^-/nm^2$ soit compris entre 4 et 10.

3 - Silice selon l'une des revendications 1 et 2 caractérisée par le fait que son PZC est compris entre 3 et 6,5.

4 - Silice selon l'une des revendications 1 à 3 caractérisée par le fait qu'elle présente une compatibilité avec les cations métalliques bivalents et plus choisis dans les groupes 2a, 3a, 4a et 8 de la classification périodique d'au moins 30 %, de préférence d'au moins 50 % et plus particulièrement d'au moins 80 %.

5 - Silice selon la revendication 4 caractérisée par le fait que le cation métallique est le calcium, le strontium, le baryum, l'aluminium, l'indium, le germanium, l'étain, le plomb, le manganèse, le fer, le nickel, le zinc, le titane, le zirconium, le palladium.

6 - Silice selon l'une des revendications 4 et 5 caractérisée par le fait que le cation métallique est sous forme de sels minéraux, chlorure, fluorure, nitrate, phosphate, sulfate ou sous forme de sels organiques acétate, citrate.

7 - Silice selon la revendication 5 caractérisée par le fait que le cation métallique est sous forme de citrate de zinc, de sulfate de zinc, de chlorure de strontium, de fluorure d'étain.

8 - Silice selon la revendication 1 caractérisée par le fait qu'elle présente une compatibilité avec l'anion fluorure d'au moins 80 % et, de préférence, d'au moins 90 %.

9 - Silice selon l'une des revendicaitons 1 à 8 caractérisée par le fait qu'elle présente une teneur en cations métalliques bivalents et plus d'au plus 1000 ppm.

10 - Silice selon la revendication 9 caractérisée par le fait que la teneur en aluminium est d'au plus 500 ppm, la teneur en fer d'au plus 200 ppm, la teneur en calcium d'au plus 500 ppm et, plus particulièrement, d'au plus 300 ppm.

11 - Silice selon l'une des revendications 1 à 10 caractérisée par le fait qu'elle présente une teneur en carbone d'au plus 50 ppm et plus particulièrement d'au plus 10 ppm.

12 - Silice selon l'une des revendications 1 à 11 caractérisée par le fait qu'elle présente un pH d'au plus 7,0 et plus particulièrement compris entre 6,0 et 7,0.

13 - Silice selon l'une des revendications 1 à 12 caractérisée par le fait qu'elle présente une surface BET comprise entre 40 et 600 m²/g.

14 - Silice selon l'une des revendications 1 à 13 caractérisée par le fait qu'elle présente une surface CTAB comprise entre 40 et 400 m²/g.

15 - Silice selon l'une des revendications 1 à 14 caractérisée par le fait qu'elle présente une surface BET comprise entre 40 et 300 m²/g.

16 - Silice selon la revendication 15 caractérisée par le fait qu'elle présente une surface CTAB comprise entre 40 et 100 m²/g.

17 - Silice selon l'une quelconque des revendications 1 à 14 du type épaississant caractérisée par le fait qu'elle présente une surface BET comprise entre 120 et 450 m²/g plus particulièrement 120 et 200 m²/g.

18 - Silice selon la revendication 17 caractérisée par le fait qu'elle présente une surface CTAB comprise entre 120 et 400 m²/g.

19 - Silice selon l'une des revendications 1 à 14 du type bifonctionnelle, caractérisée par le fait qu'elle présente une surface BET comprise entre 80 et 200 m²/g.

20 - Silice selon la revendication 19 caractérisée par le fait qu'elle présente une surface CTAB comprise entre 80 et 200 m²/g.

21 - Silice selon l'une des revendications 1 à 20 caractérisée par le fait qu'elle présente une prise d'huile comprise entre 80 et 500 cm³/100g.

22 - Silice selon l'une des revendications 1 à 21 caractérisée par le fait qu'elle présente une taille moyenne des particules comprise entre 1 et 10 μm.

23 - Silice selon l'une des revendications 1 à 22 caractérisée par le fait qu'elle présente une densité apparente comprise entre 0,01 et 0,3.

24 - Silice selon l'une des revendications 1 à 23 caractérisée par le fait qu'il s'agit d'une silice de précipitation.

25 - Procédé de préparation de la silice décrite dans l'une des revendications 1 à 24 caractérisé par le fait qu'il consiste à faire réagir un silicate avec un acide conduisant ainsi à une suspension ou un gel de silice, à effectuer un premier mûrissement à un pH supérieur ou égal à 6 et inférieur ou égal à 8,5, puis un deuxième mûrissement à un pH inférieur ou égal à 5,0 ; à séparer la silice ; à la soumettre à un lavage à l'eau chaude jusqu'à ce qu'il conduise à une suspension aqueuse dont le pH mesuré sur une suspension à 20 % de $SiO_2$ réponde à l'équation suivante :

$$pH = d - c \log (D) \quad (II)$$

dans l'équation (II) :

. c est une constante inférieure ou égale à 1,0,

. d est une constante inférieure ou égale à 8,5,

. (D) représente la conductivité électrique de la suspension aqueuse de silice exprimée en microsiemens.$cm^{-1}$ ;

enfin à la sécher.

26 - Procédé selon la revendication 25 caractérisé par le fait qu'il consiste à préparer la suspension ou le gel de silice en ajoutant simultanément le silicate et l'acide sur un pied de cuve qui peut-être de l'eau, une dispersion colloïdale de silice contenant de 0 à 150 g/l de silice exprimée en $SiO_2$, un silicate ou un sel minéral ou organique, de préférence de métal alcalin.

27 - Procédé selon la revendication 25 caractérisé par le fait que l'addition des deux réactifs se fait de manière simultanée de telle sorte que le pH soit maintenu constant entre 4 et 10, de préférence entre 8,5 et 9,5.

28 - Procédé selon l'une des revendications 25 et 26 caractérisé par le fait que la température est comprise entre 60°C et 95°C.

29 - Procédé selon la revendication 25 caractérisé par le fait que l'on prépare la dispersion colloïdale de silice contenant de 20 à 150 g/l de silice en chauffant une solution aqueuse de silicate entre 60°C et 95°C et à ajouter l'acide dans ladite solution aqueuse jusqu'à obtention d'un pH compris entre 8,0 et 10,0, de préférence égal à 9,5.

30 - Procédé selon l'une des revendications 25 à 29 caractérisé par le fait que l'on effectue un premier mûrissement de la suspension ou du gel de silice à un pH compris entre 6 et 8,5, de préférence égal à 8,0 à une température comprise entre 60°C et 100°C, de préférence égale à 95°C.

31 - Procédé selon la revendication 25 caractérisé par le fait qu'il consiste à préparer une suspension ou un gel de silice en ajoutant progressivement l'acide dans un pied de cuve contenant le silicate jusqu'à obtention du pH de mûrissement désiré à une température comprise entre 60°C et 95°C.

32 - Procédé selon la revendication 25 caractérisé par le fait que l'on effectue un lavage à l'eau ou à l'aide d'une solution acide ayant une température comprise entre 40°C et 80°C, de préférence entre 60°C et 80°C.

33 - Procédé selon la revendication 32 caractérisé par le fait que la solution acide précité est une solution d'un acide organique notamment d'un acide complexant.

34 - Procédé selon la revendication 33 caractérisé par le fait que l'acide organique précité est choisi dans le groupe des acides carboxyliques, des acides dicarboxyliques, des acides aminocarboxyliques, des acides hydrocarboxyliques.

35 - Procédé selon l'une des revendications 33 et 34 caractérisé par le fait que l'acide organique est choisi dans le groupe comprenant les acides acétique, gluconique, tartrique, citrique, maléique et glycérique.

36 - Composition dentifrice caractérisée par le fait qu'elle contient une silice selon l'une des revendications 1 à 24 ou une silice préparée par le procédé selon l'une des revendications 25 à 35.

37 - Composition dentifrice selon la revendication 36 caractérisée par le fait qu'elle comprend au moins un élément choisi dans le groupe comprenant le fluor, les phosphates.

38 - Composition dentifrice selon la revendication 36 caractérisée par le fait qu'elle comprend au moins un cation métallique bivalent et plus choisi dans le groupe 2a, 3a, 4a et 8 de la classification périodique.

39 - Composition dentifrice selon la reveridication 36 caractérisée par le fait que le cation métallique est le calcium, le strontium, le baryum, l'aluminium, l'indium, le germanium, l'étain, le plomb, le manganèse, le fer, le nickel, le zinc, le titane, le zirconium, le palladium.

40 - Composition dentifrice selon l'une des revendications 36 et 37 caractérisée par le fait que le cation métallique est sous forme de sels minéraux, chlorure, fluorure, nitrate, phosphate, sulfate ou sous forme de sels organiques acétate, citrate.

41 - Composition dentifrice selon l'une des revendications 36 à 40 caractérisée par le fait que le cation métallique est sous forme de citrate de zinc, de sulfate de zinc, de chlorure de strontium, de fluorure d'étain.

## Revendications pour l'Etat contractant suivant : ES

1 - Procédé de préparation d'une silice, cette silice présentant une chimie de surface telle que le nombre d'$OH^-$ exprimé en $OH^-/nm^2$ est inférieur ou égal à 10, que son point de charge nulle (PZC) soit compris entre 3 et 6,5 et conduisant à une suspension aqueuse dont le pH varie en fonction de sa conductivité électrique selon l'équation suivante (I) :

$$pH = b - a \log (D) \quad (I)$$

dans l'équation (I) :

. a est une constante inférieure ou égale à 0,6,

. b est une constante inférieure ou égale à 8,5,

D) représente la conductivité électrique de la suspension aqueuse de silice exprimée en microsiemens.$cm^{-1}$,

caractérisé par le fait qu'il consiste à faire réagir un silicate avec un acide conduisant ainsi à une suspension ou un gel de silice, à effectuer un premier mûrissement à un pH supérieur ou égal à 6 et inférieur ou égal à 8,5, puis un deuxième mûrissement à un pH inférieur ou égal à 5,0; à séparer la silice; à la soumettre à un lavage à l'eau chaude jusqu'à ce qu'il conduise à une suspension aqueuse dont le pH mesuré sur une suspension à 20 % de $SiO_2$ réponde à l'équation suivante:

$$pH = d - c \log (D) \quad (II)$$

dans l'équation (II) :

. c est une constante inférieure ou égale à 1,0,

. d est une constante inférieure ou égale à 8,5,

. (D) représente la conductivité électrique de la suspension aqueuse de silice exprimée en microsiemens.$cm^{-1}$ ;

enfin à la sécher.

2 - Procédé selon la revendication 1 caractérisé par le fait que la silice présente une chimie de surface telle que le nombre de $OH^-$ exprimé en $OH^-/nm^2$ soit compris entre 4 et 10.

3 - Procédé selon l'une des revendications 1 et 2 caractérisé par le fait que la silice présente un PZC compris entre 3 et 6,5.

4 - Procédé selon l'une des revendications 1 à 3 caractérisé par le fait que la silice présente une compatibilité avec les cations métalliques bivalents et plus choisis dans les groupes 2a, 3a, 4a et 8 de la classification périodique d'au moins 30 %, de préférence d'au moins 50 % et plus particulièrement d'au moins 80 %.

5 - Procédé selon la revendication 4 caractérisé par le fait que le cation métallique est le calcium, le strontium, le baryum, l'aluminium, l'indium, le germanium, l'étain, le plomb, le manganèse, le fer, le nickel, le zinc, le titane, le zirconium, le palladium.

6 - Procédé selon l'une des revendications 4 et 5 caractérisé par le fait que le cation métallique est sous forme de sels minéraux, chlorure, fluorure, nitrate, phosphate, sulfate ou sous forme de sels organiques acétate, citrate.

7 - Procédé selon la revendication 5 caractérisé par le fait que le cation métallique est sous forme de citrate de zinc, de sulfate de zinc, de chlorure de strontium, de fluorure d'étain.

8 - Procédé selon la revendication 1 caractérisé par le fait que la silice présente une compatibilité avec l'anion fluorure d'au moins 80 % et, de préférence, d'au moins 90 %.

9 - Procédé selon l'une des revendicaitons 1 à 8 caractérisé par le fait que la silice présente une teneur en cations métalliques bivalents et plus d'au plus 1000 ppm.

10 - Procédé selon la revendication 9 caractérisé par le fait que la teneur en aluminium est d'au plus 500 ppm, la teneur en fer d'au plus 200 ppm, la teneur en calcium d'au plus 500 ppm et, plus particulièrement, d'au plus 300 ppm.

11 - Procédé selon l'une des revendications 1 à 10 caractérisé par le fait que la silice présente une teneur en carbone d'au plus 50 ppm et plus particulièrement d'au plus 10 ppm.

12 - Procédé selon l'une des revendications 1 à 11 caractérisé par le fait que la silice présente un pH d'au plus 7,0 et plus particulièrement compris entre 6,0 et 7,0.

13 - Procédé selon l'une des revendications 1 à 12 caractérisé par le fait que la silice présente une surface BET comprise entre 40 et 600 m²/g.

14 - Procédé selon l'une des revendications 1 à 13 caractérisé par le fait que la silice présente une surface CTAB comprise entre 40 et 400 m²/g.

15 - Procédé selon l'une des revendications 1 à 14 caractérisé par le fait que la silice présente une surface BET comprise entre 40 et 300 m²/g.

16 - Procédé selon la revendication 15 caractérisé par le fait que la silice présente une surface CTAB comprise entre 40 et 100 m²/g.

17 - Procédé selon l'une quelconque des revendications 1 à 14 caractérisé par le fait que la silice est du type épaississante et présente une surface BET comprise entre 120 et 450 m²/g, plus particulièrement 120 et 200 m²/g.

18 - Procédé selon la revendication 17 caractérisé par le fait que la silice présente une surface CTAB comprise entre 120 et 400 m²/g.

19 - Procédé selon l'une des revendications 1 à 14, caractérisé par le fait que la silice est du type bifonctionnelle et présente une surface BET comprise entre 80 et 200 m²/g.

20 - Procédé selon la revendication 19 caractérisé par le fait que la silice présente une surface CTAB comprise entre 80 et 200 m²/g.

21 - Procédé selon l'une des revendications 1 à 20 caractérisé par le fait que la silice présente une prise d'huile comprise entre 80 et 500 cm³/100g.

22 - Procédé selon l'une des revendications 1 à 21 caractérisé par le fait que la silice présente une taille moyenne des particules comprise entre 1 et 10 μm.

23 - Procédé selon l'une des revendications 1 à 22 caractérisé par le fait que la silice présente une densité apparente comprise entre 0,01 et 0,3.

24 - Procédé selon l'une des revendications 1 à 23 caractérisé par le fait que la silice est une silice de précipitation.

25 - Procédé selon l'une des revendications 1 à 24 caractérisé par le fait qu'il consiste à préparer la suspension ou le gel de silice en ajoutant simultanément le silicate et l'acide sur un pied de cuve qui peut-être de l'eau, une dispersion colloïdale de silice contenant de 0 à 150 g/l de silice exprimée en $SiO_2$, un silicate ou un sel minéral ou organique, de préférence de métal alcalin.

26 - Procédé selon l'une des revendications 1 à 24 caractérisé par le fait que l'addition des deux réactifs se fait de manière simultanée de telle sorte que le pH soit maintenu constant entre 4 et 10, de préférence entre 8,5 et 9,5.

27 - Procédé selon l'une des revendications 1 à 25 caractérisé par le fait que la température est comprise entre 60°C et 95°C.

28 - Procédé selon l'une des revendications 1 à 24 caractérisé par le fait que l'on prépare la dispersion colloïdale de silice contenant de 20 à 150 g/l de silice en chauffant une solution aqueuse de silicate entre 60°C et 95°C et à ajouter l'acide dans ladite solution aqueuse jusqu'à obtention d'un pH compris entre 8,0 et 10,0, de préférence égal à 9, 5.

29 - Procédé selon l'une des revendications 1 à 28 caractérisé par le fait que l'on effectue un premier mûrissement de la suspension ou du gel de silice à un pH compris entre 6 et 8,5, de préférence égal à 8,0 à une température comprise entre 60°C et 100°C, de préférence égale à 95°C.

30 - Procédé selon l'une des revendications 1 à 30 caractérisé par le fait qu'il consiste à préparer une suspension ou un gel de silice en ajoutant progressivement l'acide dans un pied de cuve contenant le silicate jusqu'à obtention du pH de mûrissement désiré à une température comprise entre 60°C et 95°C.

31 - Procédé selon l'une des revendications 1 à 24 caractérisé par le fait que l'on effectue un lavage à l'eau ou à l'aide d'une solution acide ayant une température comprise entre 40°C et 80°C, de préférence entre 60°C et 80°C.

32 - Procédé selon la revendication 31 caractérisé par le fait que la solution acide précité est une solution d'un acide organique notamment d'un acide complexant.

33 - Procédé selon la revendication 32 caractérisé par le fait que l'acide organique précité est choisi dans le groupe des acides carboxyliques, des acides dicarboxyliques, des acides aminocarboxyliques, des acides hydrocarboxyliques.

34 - Procédé selon l'une des revendications 32 et 33 caractérisé par le fait que l'acide organique est choisi dans le groupe comprenant les acides acétique, gluconique, tartrique, citrique, maléique et glycérique.

35 - Composition dentifrice caractérisée par le fait qu'elle contient une silice préparée par le procédé selon l'une des revendications 1 à 34.

36 - Composition dentifrice selon la revendication 35 caractérisée par le fait qu'elle comprend au moins un élément choisi dans le groupe comprenant le fluor, les phosphates.

37 - Composition dentifrice selon la revendication 35 caractérisée par le fait qu'elle comprend au moins un cation métallique bivalent et plus choisi dans le groupe 2a, 3a, 4a et 8 de la classification périodique.

38 - Composition dentifrice selon la revendication 35 caractérisée par le fait que le cation métallique est le calcium, le strontium, le baryum, l'aluminium, l'indium, le germanium, l'étain, le plomb, le manganèse, le fer, le nickel, le zinc, le titane, le zirconium, le palladium.

39 - Composition dentifrice selon l'une des revendications 35 et 36 caractérisée par le fait que le cation métallique est sous forme de sels minéraux, chlorure, fluorure, nitrate, phosphate, sulfate ou sous forme de sels organiques acétate, citrate.

40 - Composition dentifrice selon l'une des revendications 35 à 39 caractérisée par le fait que le cation métallique est sous forme de citrate de zinc, de sulfate de zinc, de chlorure de strontium, de fluorure d'étain.

**Patentansprüche**

**Patentansprüche für folgende Vertragsstaaten : AT, BE, CH, DE, DK, FR, GB, GR, IT, LI, LU, NL, SE**

1. Kieselsäure, dadurch gekennzeichnet, daß sie eine solche Oberflächenchemie aufweist, daß die Anzahl von $OH^-$ ausgedrückt in $OH^-/nm^2$ kleiner oder gleich 10 ist, ihr Ladungspunkt Null (PZC) zwischen 3 und 6,5 liegt und daß sie zu einer wäßrigen Suspension führt, deren pH-Wert gemäß ihrer elektrischen Leitfähigkeit nach der folgenden Gleichung (I):

$$pH = b - a \log (D) \quad (I)$$

variiert, wobei in der Gleichung (I)
   – a eine Konstante kleiner oder gleich 0,6,
   – b eine Konstante kleiner oder gleich 8,5,
   – (D) die elektrische Leitfähigkeit der wäßrigen Kieselsäuresuspension ausgedrückt in Mikrosiemens $cm^{-1}$ bedeuten.

2. Kieselsäure nach Anspruch 1, dadurch gekennzeichnet, daß sie eine solche Oberflächenchemie aufweist, daß die Anzahl von $OH^-$ ausgedrückt in $OH^-/nm^2$ zwischen 4 und 10 liegt.

3. Kieselsäure nach einem der Ansprüche 1 und 2, dadurch gekennzeichnet, daß ihr PZC zwischen 3 und 6,5 liegt.

4. Kieselsäure nach einem der Ansprüche 1 und 3, dadurch gekennzeichnet, daß sie eine Verträglichkeit mit den zwei- und mehrwertigen Metallkationen ausgewählt aus den Gruppen 2a, 3a, 4a und 8 des periodischen Systems von mindestens 30 %, vorzugsweise von mindestens 50 % und insbesondere von mindestens 80 % aufweist.

5. Kieselsäure nach Anspruch 4, dadurch gekennzeichnet, daß das Metallkation Calcium, Strontium, Barium, Aluminium, Indium, Germanium, Zinn, Blei, Mangan, Eisen, Nickel, Zink, Titan, Zirkonium oder Palladium ist.

6. Kieselsäure nach einem der Ansprüche 4 und 5, dadurch gekennzeichnet, daß das Metallkation in form der anorganischen Salze Chlorid, fluorid, Nitrat, Phosphat, Sulfat oder in form der organischen Salze Acetat, Citrat vorliegt.

7. Kieselsäure nach Anspruch 5, dadurch gekennzeichnet, daß das Metallkation als Zinkcitrat, Zinksulfat, Strontiumchlorid oder Zinnfluorid vorliegt.

8. Kieselsäure nach Anspruch 1, dadurch gekennzeichnet, daß sie eine Verträglichkeit mit dem Fluoridanion von mindestens 80 % und vorzugsweise von mindestens 90 % aufweist.

9. Kieselsäure nach einem der Ansprüche 1 bis 8, dadurch gekennzeichnet, daß sie einen Gehalt an zwei- und mehrwertigen Metallkationen von höchstens 1000 ppm aufweist.

10. Kieselsäure nach Anspruch 9, dadurch gekennzeichnet, daß der Aluminiumgehalt höchstens 500 ppm, der Eisengehalt höchstens 200 ppm, der Calciumgehalt höchstens 500 ppm und insbesondere höchstens 300 ppm ist.

11. Kieselsäure nach einem der Ansprüche 1 bis 10, dadurch gekennzeichnet, daß sie einen Kohlenstoffgehalt von höchstens 50 ppm und insbesondere von höchstens 10 ppm aufweist.

12. Kieselsäure nach einem der Ansprüche 1 bis 11, dadurch gekennzeichnet, daß sie einen pH-Wert von höchstens 7,0 und insbesondere zwischen 6,0 und 7,0 aufweist.

13. Kieselsäure nach einem der Ansprüche 1 bis 12, dadurch gekennzeichnet, daß sie eine BET-Oberfläche zwischen 40 und 600 $m^2/g$ aufweist.

14. Kieselsäure nach einem der Ansprüche 1 bis 13, dadurch gekennzeichnet, daß sie eine CTAB-Oberfläche zwischen 40 und 400 $m^2/g$ aufweist.

15. Kieselsäure nach einem der Ansprüche 1 bis 14, dadurch gekennzeichnet, daß sie eine BET-Oberfläche zwischen 40 und 300 $m^2/g$ aufweist.

16. Kieselsäure nach Anspruch 15, dadurch gekennzeichnet, daß sie eine CTAB-Oberfläche zwischen 40 und 100 $m^2/g$ aufweist.

17. Kieselsäure nach einem der Ansprüche 1 bis 14 vom Typ Verdickungsmittel, dadurch gekennzeichnet, daß sie eine BET-Oberfläche zwischen 120 und 450 $m^2/g$, insbesondere zwischen 120 und 200 $m^2/g$ aufweist.

18. Kieselsäure nach Anspruch 17, dadurch gekennzeichnet, daß sie eine CTAB-Oberfläche zwischen 120 und 400 $m^2/g$ aufweist.

19. Kieselsäure nach einem der Ansprüche 1 bis 14 vom bifunktionellen Typ, dadurch gekennzeichnet, daß sie eine BET-Oberfläche zwischen 80 und 200 $m^2/g$ aufweist.

20. Kieselsäure nach Anspruch 19, dadurch gekennzeichnet, daß sie eine CTAB-Oberfläche zwischen 80 und 200 $m^2/g$ aufweist.

21. Kieselsäure nach einem der Ansprüche 1 bis 20, dadurch gekennzeichnet, daß sie eine Ölaufnahme zwischen 80 und 500 $cm^3/100$ g aufweist.

22. Kieselsäure nach einem der Ansprüche 1 bis 21, dadurch gekennzeichnet, daß sie eine mittlere Teilchengröße zwischen 1 und 10 µm aufweist.

23. Kieselsäure nach einem der Ansprüche 1 bis 22, dadurch gekennzeichnet, daß sie eine scheinbare Dichte zwischen 0,01 und 0,3 aufweist.

**24.** Kieselsäure nach einem der Ansprüche 1 bis 23, dadurch gekennzeichnet, daß es sich um eine Fällungskieselsäure handelt.

**25.** Verfahren zur Herstellung einer in den Ansprüchen 1 bis 24 beschriebenen Kieselsäure, dadurch gekennzeichnet, daß es darin besteht, daß man ein Silikat mit einer Säure zur Bildung einer Kieselsäuresuspension oder eines Kieselgels reagieren läßt, eine erste Reifung bei einem pH-Wert größer oder gleich 6 und kleiner oder gleich 8,5 ausführt, dann eine zweite Reifung bei einem pH-Wert kleiner oder gleich 5,0 ausführt, die Kieselsäure abtrennt und einer Wäsche mit warmem Wasser unterwirft bis eine wäßrige Suspension, deren pH-Wert, gemessen an einer Suspension bei 20 % $SiO_2$, der folgenden Gleichung

$$pH = d - c \log (D) \quad (II)$$

entspricht, wobei in der Gleichung (II)
  – c eine Konstante kleiner oder gleich 1,0,
  – d eine Konstante kleiner oder gleich 8,5,
  – (D) eine elektrische Leitfähigkeit der wäßrigen Kieselsäuresuspension bemessen in Mikrosiemens $cm^{-1}$ bedeuten,
und schließlich trocknet.

**26.** Verfahren nach Anspruch 25, dadurch gekennzeichnet, daß man die Kieselsäuresuspension oder das Kieselgel durch gleichzeitige Zugabe des Silikates und der Säure in eine Vorlage herstellt, die Wasser, eine kolloidale Kieselsäuredispersion enthaltend 0 bis 150 g/l Kieselsäure in Form von $SiO_2$, ein Silikat oder ein anorganisches oder organisches Salz, vorzugsweise eines Alkalimetalls, sein kann.

**27.** Verfahren nach Anspruch 25, dadurch gekennzeichnet, daß die Zugabe der zwei Reagenzien in der Weise gleichzeitig ausgeführt wird, daß der pH-Wert konstant zwischen 4 und 10, vorzugsweise zwischen 8,5 und 9,5 gehalten wird.

**28.** Verfahren nach einem der Ansprüche 25 und 26, dadurch gekennzeichnet, daß die Temperatur im Bereich zwischen 60°C und 95°C liegt.

**29.** Verfahren nach Anspruch 25, dadurch gekennzeichnet, daß man die 20 bis 150 g/l Kieselsäure enthaltende kolloidale Kieselsäuredispersion herstellt, indem man eine wäßrige Silikatlösung auf 60°C bis 95°C erhitzt und die Säure in die genannte wäßrige Lösung bis zum Erhalt eines pH-Wertes zwischen 8,0 und 10,0, vorzugsweise gleich 9,5, zugibt.

**30.** Verfahren nach einem der Ansprüche 25 bis 29, dadurch gekennzeichnet, daß man eine erste Reifung der Kieselsäuresuspension oder des Kieselgels bei einem pH-Wert zwischen 6 und 8,5, vorzugsweise bei 8,0, bei einer Temperatur zwischen 60°C und 100°C, vorzugsweise bei 95°C, durchführt.

**31.** Verfahren nach Anspruch 25, dadurch gekennzeichnet, daß es darin besteht, daß man eine Kieselsäuresuspension oder ein Kieselgel herstellt, indem man die Säure allmählich in die das Silikat enthaltende Vorlage bis zum Erhalt des für die Reifung erwünschten pH-Wertes bei einer Temperatur im Bereich zwischen 60°C und 95°C hinzufügt.

**32.** Verfahren nach Anspruch 25, dadurch gekennzeichnet, daß man eine Wäsche mit Wasser oder mit Hilfe einer sauren Lösung durchführt, die eine Temperatur im Bereich zwischen 40°C und 80°C, vorzugsweise zwischen 60°C und 80°C hat.

**33.** Verfahren nach Anspruch 32, dadurch gekennzeichnet, daß die erwähnte saure Lösung eine Lösung einer organischen Säure, insbesondere einer komplexbildenden Säure ist.

**34.** Verfahren nach Anspruch 33, dadurch gekennzeichnet, daß die erwähnte saure Lösung aus der Gruppe der Carbonsäuren, Dicarbonsäuren, Aminocarbonsäuren und Hydrocarbonsäuren ausgewählt wird.

**35.** Verfahren nach einem der Ansprüche 33 und 34, dadurch gekennzeichnet, daß die organische Säure aus der Gruppe umfassend Essigsäure, Gluconsäure, Weinsäure, Citronensäure, Maleinsäure und Glycerinsäure ausgewählt wird.

**36.** Zahnpflegemittelzusammensetzung, dadurch gekennzeichnet, daß sie eine Kieselsäure nach einem der Ansprüche 1 bis 24 oder eine gemäß dem Verfahren nach einem der Ansprüche 25 bis 35 hergestellte Kieselsäure enthält.

**37.** Zahnpflegemittelzusammensetzung nach Anspruch 36, dadurch gekennzeichnet, daß sie mindestens ein Element ausgewählt aus der Gruppe umfassend Fluor und Phosphate enthält.

**38.** Zahnpflegemittelzusammensetzung nach Anspruch 36, dadurch gekennzeichnet, daß sie mindestens ein zwei- oder mehrwertiges Metallkation ausgewählt aus den Gruppen 2a, 3a, 4a und 8 des periodischen Systems enthält.

**39.** Zahnpflegemittelzusammensetzung nach Anspruch 36, dadurch gekennzeichnet, daß das Metallkation Calcium, Strontium, Barium, Aluminium, Indium, Germanium, Zinn, Blei, Mangan, Eisen, Nickel, Zink, Titan, Zirkonium oder Palladium ist.

**40.** Zahnpflegemittelzusammensetzung nach einem der Ansprüche 36 und 37, dadurch gekennzeichnet, daß das Metallkation in form der anorganischen Salze Chlorid, fluorid, Nitrat, Phosphat, Sulfat oder in form der organischen Salze Acetat, Citrat vorliegt.

**41.** Zahnpflegemittelzusammensetzung nach einem der Ansprüche 36 bis 40, dadurch gekennzeichnet, daß das Metallkation als Zinkcitrat, Zinksulfat, Strontiumchlorid oder Zinnfluorid vorliegt.

**Patentansprüche für folgenden Vertragsstaat : ES**

**1.** Verfahren zur Herstellung einer Kieselsäure, welche eine solche Oberflächenchemie aufweist, daß die Anzahl von $OH^-$ ausgedrückt in $OH^-/nm^2$ kleiner oder gleich 10 ist, der Ladungspunkt Null (PZC) zwischen 3 und 6,5 liegt, und welche zu einer wäßrigen Suspension führt, deren pH-Wert gemäß ihrer elektrischen Leitfähigkeit nach der folgenden Gleichung (I):
$$pH = b - a \log (D) \quad (I)$$
variiert, wobei in der Gleichung (I)
  – a eine Konstante kleiner oder gleich 0,6,
  – b eine Konstante kleiner oder gleich 8,5,
  – (D) die elektrische Leitfähigkeit der wäßrigen Kieselsäuresuspension ausgedrückt in Mikrosiemens $cm^{-1}$
bedeuten, dadurch gekennzeichnet, daß es darin besteht, daß man ein Silikat mit einer Säure zur Bildung einer Kieselsäuresuspension oder eines Kieselgels reagieren läßt, eine erste Reifung bei einem pH-Wert höher oder gleich 6 und kleiner oder gleich 8,5 ausführt, dann eine zweite Reifung bei einem pH-Wert kleiner oder gleich 5,0 ausführt, die Kieselsäure abtrennt, einer Wäsche mit warmem Wasser unterwirft bis eine wäßrige Suspension, deren pH-Wert, gemessen an einer Suspension bei 20 % $SiO_2$, der folgenden Gleichung
$$pH = d - c \log (D) \quad (II)$$
entspricht, wobei in der Gleichung (II)
  – c eine Konstante kleiner oder gleich 1,0,
  – d eine Konstante kleiner oder gleich 8,5,
  – (D) die elektrische Leitfähigkeit der wäßrigen Kieselsäuresuspension bemessen in Mikrosiemens $cm^{-1}$ bedeuten,
und schließlich trocknet.

**2.** Verfahren nach Anspruch 1, dadurch gekennzeichnet, daß die Kieselsäure eine solche Oberflächenchemie aufweist, daß die Anzahl von $OH^-$ ausgedrückt in $OH^-/nm^2$ zwischen 4 und 10 liegt.

**3.** Verfahren nach einem der Ansprüche 1 und 2, dadurch gekennzeichnet, daß die Kieselsäure einen Ladungspunkt Null (PZC) zwischen 3 und 6,5 aufweist.

**4.** Verfahren nach einem der Ansprüche 1 und 3, dadurch gekennzeichnet, daß die Kieselsäure eine Verträglichkeit mit den zwei- und mehrwertigen Metallkationen ausgewählt aus den Gruppen 2a, 3a, 4a und 8 des periodischen Systems von mindestens 30 %, vorzugsweise von mindestens 50 % und insbesondere von mindestens 80 % aufweist.

**5.** Verfahren nach Anspruch 4, dadurch gekennzeichnet, daß das Metallkation Calcium, Strontium, Barium, Aluminium, Indium, Germanium, Zinn, Blei, Mangan, Eisen, Nickel, Zink, Titan, Zirkonium oder Palladium ist.

6. Verfahren nach einem der Ansprüche 4 und 5, dadurch gekennzeichnet, daß das Metallkation in form der anorganischen Salze Chlorid, fluorid, Nitrat, Phosphat, Sulfat oder in form der organischen Salze Acetat, Citrat vorliegt.

7. Verfahren nach Anspruch 5, dadurch gekennzeichnet, daß das Metallkation als Zinkcitrat, Zinksulfat, Strontiumchlorid oder Zinnfluorid vorliegt.

8. Verfahren nach Anspruch 1, dadurch gekennzeichnet, daß die Kieselsäure eine Verträglichkeit mit dem Fluoridanion von mindestens 80 %, und vorzugsweise von mindestens 90 % aufweist.

9. Verfahren nach einem der Ansprüche 1 bis 8, dadurch gekennzeichnet, daß die Kieselsäure einen Gehalt an zwei- und mehrwertigen Metallkationen von höchstens 1000 ppm aufweist.

10. Verfahren nach Anspruch 9, dadurch gekennzeichnet, daß der Aluminiumgehalt höchstens 500 ppm, der Eisengehalt höchstens 200 ppm, der Calciumgehalt höchstens 500 ppm und insbesondere höchstens 300 ppm ist.

11. Verfahren nach einem der Ansprüche 1 bis 10, dadurch gekennzeichnet, daß die Kieselsäure einen Kohlenstoffgehalt von höchstens 50 ppm und insbesondere von höchstens 10 ppm aufweist.

12. Verfahren nach einem der Ansprüche 1 bis 11, dadurch gekennzeichnet, daß die Kieselsäure einen pH-Wert von höchstens 7,0 und insbesondere zwischen 6,0 und 7,0 aufweist.

13. Verfahren nach einem der Ansprüche 1 bis 12, dadurch gekennzeichnet, daß die Kieselsäure eine BET-Oberfläche zwischen 40 und 600 m$^2$/g aufweist.

14. Verfahren nach einem der Ansprüche 1 bis 13, dadurch gekennzeichnet, daß die Kieselsäure eine CTAB-Oberfläche zwischen 40 und 400 m$^2$/g aufweist.

15. Verfahren nach einem der Ansprüche 1 bis 14, dadurch gekennzeichnet, daß die Kieselsäure eine BET-Oberfläche zwischen 40 und 300 m$^2$/g aufweist.

16. Verfahren nach Anspruch 15, dadurch gekennzeichnet, daß die Kieselsäure eine CTAB-Oberfläche zwischen 40 und 100 m$^2$/g aufweist.

17. Verfahren nach einem der Ansprüche 1 bis 14, dadurch gekennzeichnet, daß die Kieselsäure vom Typ Verdickungsmittel eine BET-Oberfläche zwischen 120 und 450 m$^2$/g, insbesondere zwischen 120 und 200 m$^2$/g aufweist.

18. Verfahren nach Anspruch 17, dadurch gekennzeichnet, daß die Kieselsäure eine CTAB-Oberfläche zwischen 120 und 400 m$^2$/g aufweist.

19. Verfahren nach einem der Ansprüche 1 bis 14, dadurch gekennzeichnet, daß die Kieselsäure vom bifunktionellen Typ eine BET-Oberfläche zwischen 80 und 200 m$^2$/g aufweist.

20. Verfahren nach Anspruch 19, dadurch gekennzeichnet, daß die Kieselsäure eine CTAB-Oberfläche zwischen 80 und 200 m$^2$/g aufweist.

21. Verfahren nach einem der Ansprüche 1 bis 20, dadurch gekennzeichnet, daß die Kieselsäure eine Ölaufnahme zwischen 80 und 500 cm$^3$/100 g aufweist.

22. Verfahren nach einem der Ansprüche 1 bis 21, dadurch gekennzeichnet, daß die Kieselsäure eine mittlere Teilchengröße zwischen 1 und 10 µm aufweist.

23. Verfahren nach einem der Ansprüche 1 bis 22, dadurch gekennzeichnet, daß die Kieselsäure eine scheinbare Dichte zwischen 0,01 und 0,3 aufweist.

24. Verfahren nach einem der Ansprüche 1 bis 23, dadurch gekennzeichnet, daß die Kieselsäure eine Fällungskieselsäure ist.

25. Verfahren nach einem der Ansprüche 1 bis 24, dadurch gekennzeichnet, daß es darin besteht, daß man die Kieselsäuresuspension oder das Kieselgel durch gleichzeitige Zugabe des Silikates und der Säure in eine Vorlage herstellt, die Wasser, eine kolloidale Kieselsäuredispersion enthaltend 0 bis 150 g/l Kieselsäure in form von SiO$_2$, ein Silikat oder ein anorganisches oder organisches Salz, vorzugsweise eines Alkalimetalls, sein kann.

26. Verfahren nach einem der Ansprüche 1 bis 24, dadurch gekennzeichnet, daß die Zugabe der zwei Reagenzien in der Weise gleichzeitig ausgeführt wird, daß der pH-Wert konstant zwischen 4 und 10, vorzugsweise zwischen 8,5 und 9,5 gehalten wird.

27. Verfahren nach einem der Ansprüche 1 bis 25, dadurch gekennzeichnet, daß die Temperatur im Bereich zwischen 60°C und 95°C liegt.

28. Verfahren nach einem der Ansprüche 1 bis 24, dadurch gekennzeichnet, daß man die 20 bis 150 g/l Kieselsäure enthaltende kolloidale Kieselsäuredispersion herstellt, indem man eine wäßrige Silikatlösung auf 60°C bis 95°C erhitzt und die Säure in die genannte wäßrige Lösung bis zum Erhalt eines pH-Wertes zwischen 8,0 und 10,0, vorzugsweise gleich 9,5, zugibt.

29. Verfahren nach einem der Ansprüche 1 bis 28, dadurch gekennzeichnet, daß man eine erste Reifung der Kieselsäuresuspension oder des Kieselgels bei einem pH-Wert zwischen 6 und 8,5, vorzugsweise bei 8,0, bei einer Temperatur im Bereich zwischen 60°C und 100°C, vorzugsweise bei 95°C, durchführt.

30. Verfahren nach einem der Ansprüche 1 bis 29, dadurch gekennzeichnet, daß es darin besteht, daß man eine Kieselsäuresuspension oder ein Kieselgel herstellt, indem man die Säure allmählich in die das Silikat enthaltende Vorlage bis zum Erhalt des für die Reifung erwünschten pH-Wertes bei einer Temperatur im Bereich zwischen 60°C und 95°C hinzufügt.

31. Verfahren nach einem der Ansprüche 1 bis 24, dadurch gekennzeichnet, daß man eine Wäsche mit Wasser oder mit Hilfe einer sauren Lösung durchführt, die eine Temperatur im Bereich zwischen 40°C und 80°C, vorzugsweise 60°C und 80°C hat.

32. Verfahren nach Anspruch 31, dadurch gekennzeichnet, daß die erwähnte saure Lösung eine Lösung einer organischen Säure, insbesondere einer komplexbildenden Säure ist.

33. Verfahren nach Anspruch 32, dadurch gekennzeichnet, daß die erwähnte saure Lösung aus der Gruppe der Carbonsäuren, Dicarbonsäuren, Aminocarbonsäuren und Hydrocarbonsäuren ausgewählt wird.

34. Verfahren nach einem der Ansprüche 32 und 33, dadurch gekennzeichnet, daß die organische Säure aus der Gruppe umfassend Essigsäure, Gluconsäure, Weinsäure, Citronensäure, Maleinsäure und Glycerinsäure ausgewählt wird.

35. Zahnpflegemittelzusammensetzung, dadurch gekennzeichnet, daß sie eine gemäß dem Verfahren nach einem der Ansprüche 1 bis 34 hergestellte Kieselsäure enthält.

36. Zahnpflegemittelzusammensetzung nach Anspruch 35, dadurch gekennzeichnet, daß sie mindestens ein Element ausgewählt aus der Gruppe umfassend fluor und Phosphate enthält.

37. Zahnpflegemittelzusammensetzung nach Anspruch 35, dadurch gekennzeichnet, daß sie mindestens ein zwei- oder mehrwertiges Metallkation ausgewählt aus den Gruppen 2a, 3a, 4a und 8 des periodischen Systems enthält.

38. Zahnpflegemittelzusammensetzung nach Anspruch 35, dadurch gekennzeichnet, daß das Metallkation Calcium, Strontium, Barium, Aluminium, Indium, Germanium, Zinn, Blei, Mangan, Eisen, Nickel, Zink, Titan, Zirkonium oder Palladium ist.

39. Zahnpflegemittelzusammensetzung nach einem der Ansprüche 35 und 36, dadurch gekennzeichnet, daß das Metallkation in form der anorganischen Salze Chlorid, fluorid, Nitrat, Phosphat, Sulfat oder in form der organischen Salze Acetat, Citrat vorliegt.

40. Zahnpflegemittelzusammensetzung nach einem der Ansprüche 35 bis 39, dadurch gekennzeichnet, daß das Metallkation in form von Zinkcitrat, Zinksulfat, Strontiumchlorid oder Zinnfluorid vorliegt.

## Claims

**Claims for the following Contracting States : AT, BE, CH, DE, DK, FR, GB, GR, IT, LI, LU, NL, SE**

1.  Silica, characterized in that it has a surface chemistry such that the number of OH⁻ expressed in $OH^-/nm^2$ is equal to or below 10%, its zero charge point (ZCP) is between 3 and 6.5 and leads to an aqueous suspension, whose pH varies as a function of its electrical conductivity according to the following equation (I):

$$pH = b - a \log (D) \quad (I)$$

    in which
    a is a constant equal to or below 0.6,
    b is a constant equal to or below 8.5,
    (D) represents the electrical conductivity of the aqueous silica suspension expressed in microsiemens.cm⁻¹.

2.  Silica according to claim 1, characterized in that it has a surface chemistry such that the number of OH⁻ expressed in $OH^-/nm^2$ is between 4 and 10.

3.  Silica according to one of the claims 1 and 2, characterized in that its ZCP is between 3 and 6.5.

4.  Silica according to any one of the claims 1 to 3, characterized in that it has a compatibility with divalent and higher valency metal cations chosen from groups 2a,3a,4a and 8 of the periodic classification of at least 30%, preferably at least 50% and more particularly at least 80%.

5.  Silica according to claim 4, characterized in that the metal cation is calcium, strontium, barium, aluminium, indium, germanium, tin, lead, manganese, iron, nickel, zinc, titanium, zirconium or palladium.

6.  Silica according to one of the claims 4 and 5, characterized in that the metal cation is in the form of the mineral salts chloride, fluoride, nitrate, phosphate or sulphate or in the form of the organic salts acetate and citrate.

7.  Silica according to claim 5, characterized in that the metal cation is in the form zinc citrate, zinc sulphate, strontium chloride or tin fluoride.

8.  Silica according to claim 1, characterized in that it has a compatibility with the fluoride anion of at least 80% and preferably at least 90%.

9.  Silica according to one of the claims 1 to 8, characterized in that it has a content of divalent and higher valency metal cations of at the most 1000 ppm.

10. Silica according to claim 9, characterized in that the aluminium content is at most 500 ppm, the iron content at the most 200 ppm, the calcium content at the most 500 ppm and more particularly at the most 300 ppm.

11. Silica according to one of the claims 1 to 10, characterized in that it has a carbon content of at the most 50 ppm and more particularly at the most 10 ppm.

12. Silica according to one of the claims 1 to 11, characterized in that it has a pH of at the most 7.0 and more particularly between 6.0 and 7.0.

13. Silica according to one of the claims 1 to 12, characterized in that it has a BET surface between 40 and 600 $m^2/g$.

14. Silica according to one of the claims 1 to 13, characterized in that it has a CTAB surface between 40 and 400 $m^2/g$.

15. Silica according to one of the claims 1 to 14, characterized in that it has a BET surface between 40 and 300 $m^2/g$.

16. Silica according to claim 15, characterized in that it has a CTAB surface between 40 and 100 $m^2/g$.

17. Silica according to any one of the claims 1 to 14 of the thickening type, characterized in that it has a BET surface between 120 and 450 m$^2$/g and more particularly between 120 and 200 m$^2$/g.

18. Silica according to claim 17, characterized in that it has a CTAB surface between 120 and 400 m$^2$/g.

19. Silica according to one of the claims 1 to 14 of the bifunctional type, characterized in that it has a BET surface between 80 and 200 m$^2$/g.

20. Silica according to claim 19, characterized in that it has a CTAB surface between 80 and 200 m$^2$/g.

21. Silica according to one of the claims 1 to 20, characterized in that it has an oil absorption between 80 and 500 cm$^3$/100g.

22. Silica according to one of the claims 1 to 21, characterized in that it has a mean particle size between 1 and 10 μm.

23. Silica according to one of the claims 1 to 22, characterized in that it has an apparent density between 0.01 and 0.3.

24. Silica according to one of the claims 1 to 23, characterized in that it is a precipitation silica.

25. Process for the preparation of the silica described in one of the claims 1 to 24, characterized in that it consists of reacting a silicate with an acid, thus leading to a silica gel or a suspension, carrying out a first aging at a pH equal to or above 6 and equal to or below 8.5, then a second aging at a pH equal to or below 5, separating the silica, subjecting it to washing with hot water until it leads to an aqueous suspension, whose pH, measured on a 20% SiO$_2$ suspension, is in accordance with the following equation:

$$pH = d - c \log (D) \quad (II)$$

in which:

   c is a constant equal to or below 1.0,
   d is a constant equal to or below 8.5,
   (D) represents the electrical conductivity of the aqueous silica suspension expressed in microsiemens.cm$^{-1}$ and finally drying it.

26. Process according to claim 25, characterized in that it consists of preparing the suspension or silica gel by simultaneously adding the silicate and the acid to a sediment, which can be water, a colloidal silica dispersion containing 0 to 150 g/l of silica expressed as SiO$_2$, a silicate or a mineral or organic salt, preferably an alkali metal salt.

27. Process according to claim 25, characterized in that the addition of the two reagents takes place simultaneously in such a way that the pH is kept constant at between 4 and 10 and preferably between 8.5 and 9.5.

28. Process according to any one of the claims 25 and 26, characterized in that the temperature is between 60 and 95°C.

29. Process according to claim 25, characterized in that the colloidal silica dispersion containing 20 to 150 g/l of silica is prepared by heating an aqueous silicate solution at between 60 and 95°C and adding acid to said aqueous solution until a pH between 8.0 and 10.0 and preferably 9.5 is obtained.

30. Process according to one of the claims 25 to 29, characterized in that a first aging of the suspension or silica gel takes place at a pH between 6 and 8.5 and preferably at 8.0, at a temperature between 60 and 100°C and preferably at 95°C.

31. Process according to claim 25, characterized in that it consists of preparing a suspension or a silica gel by progressively adding acid to a sediment containing the silicate until the desired aging pH is obtained at a temperature between 60 and 95°C.

32. Process according to claim 25, characterized in that washing takes place with water or with the aid of an acid solution with a temperature between 40 and 80°C and preferably between 60 and 80°C.

33. Process according to claim 32, characterized in that the above acid solution is an organic acid solution, particularly a complexing acid.

34. Process according to claim 33, characterized in that the aforementioned organic acid is chosen from among carboxylic, dicarboxylic, aminocarboxylic and hydroxycarboxylic acids.

35. Process according to one of the claims 33 and 34, characterized in that the organic acid is chosen from among acetic, gluconic, tartaric, citric, maleic and glyceric acid.

36. Dentifrice composition, characterized in that it contains a silica according to one of the claims 1 to 24, or a silica prepared by the process according to one of the claims 25 to 35.

37. Dentifrice composition according to claim 36, characterized in that it comprises at least one element chosen from the group including fluorine and phosphates.

38. Dentifrice composition according to claim 36, characterized in that it comprises at least one divalent or higher valency metal cation chosen from group 2a,3a,4a and 8 of the periodic classification.

39. Dentifrice composition according to claim 36, characterized in that the metal cation is calcium, strontium, barium, aluminium, indium, germanium, tin, lead, manganese, iron, nickel, zinc, titanium, zirconium or palladium.

40. Dentifrice composition according to one of the claims 36 and 37, characterized in that the metal cation is in the form of mineral salts chloride, fluoride, nitrate, phosphate or sulphate, or in the form of organic salts, namely acetate or citrate.

41. Dentifrice composition according to one of the claims 36 to 40, characterized in that the metal cation is in the form zinc citrate, zinc sulphate, strontium chloride or tin fluoride.

**Claims for the following Contracting State : ES**

1. Process for the preparation of a silica having a surface chemistry such that the number of $OH^-$ expressed in $OH^-/nm^2$ is equal to or below 10%,its zero charge point (ZCP) is between 3 and 6.5 and leads to an aqueous suspension, whose pH varies as a function of its electrical conductivity according to the following equation (I):
$$pH = b - a \log (D) \quad (I)$$
in which
a is a constant equal to or below 0.6,
b is a constant equal to or below 8.5,
(D) represents the electrical conductivity of the aqueous silica suspension expressed in microsiemens.$cm^{-1}$,
characterized in that it consists of reacting a silicate with an acid, thus leading to a silica gel or a suspension, carrying out a first aging at a pH equal to or above 6 and equal to or below 8.5, then a second aging at a pH equal to or below 5, separating the silica, subjecting it to washing with hot water until it leads to an aqueous suspension whose pH, measured on a 20% $SiO_2$ suspension, is in accordance with the following equation:
$$pH = d - c \log (D) \quad (II)$$
in which
c is a constant equal to or below 1.0,
d is a constant equal to or below 8.5,
(D) represents the electrical conductivity of the aqueous silica suspension expressed in microsiemens.$cm^{-1}$ and finally drying it.

2. Process according to claim 1, characterized in that the silica has a surface chemistry such that the number of $OH^-$ expressed in $OH^-/nm^2$ is between 4 and 10.

3. Process according to one of the claims 1 and 2, characterized in that the silicas ZCP is between 3 and 6.5.

4. Process according to any one of the claims 1 to 3, characterized in that the silica has a compatibility with divalent and higher valency metal cations chosen from groups 2a, 3a, 4a and 8 of the periodic classification of at least 30%, preferably at least 50% and more particularly at least 80%.

5. Process according to claim 4, characterized in that the metal cation is calcium, strontium, barium, aluminium, indium, germanium, tin, lead, manganese, iron, nickel, zinc, titanium, zirconium or palladium.

6. Process according to one of the claims 4 and 5, characterized in that the metal cation is in the form of the mineral salts chloride, fluoride, nitrate, phosphate or sulphate or in the form of the organic salts acetate and citrate.

7. Process according to claim 5, characterized in that the metal cation is in the form zinc citrate, zinc sulphate, strontium chloride or tin fluoride.

8. Process according to claim 1, characterized in that the silica has a compatibility with the fluoride anion of at least 80% and preferably at least 90%.

9. Process according to one of the claims 1 to 8, characterized in that the silica has a content of divalent and higher valency metal cations of at the most 1000 ppm.

10. Process according to claim 9, characterized in that the aluminium content is at most 500 ppm, the iron content at the most 200 ppm, the calcium content at the most 500 ppm and more particularly at the most 300 ppm.

11. Process according to one of the claims 1 to 10, characterized in that the silica has a carbon content of at the most 50 ppm and more particularly at the most 10 ppm.

12. Process according to one of the claims 1 to 11, characterized in that the silica has a pH of at the most 7.0 and more particularly between 6.0 and 7.0.

13. Process according to one of the claims 1 to 12, characterized in that the silica has a BET surface between 40 and 600 $m^2/g$.

14. Process according to one of the claims 1 to 13, characterized in that the silica has a CTAB surface between 40 and 400 $m^2/g$.

15. Process according to one of the claims 1 to 14, characterized in that the silica has a BET surface between 40 and 300 $m^2/g$.

16. Process according to claim 15, characterized in that the silica has a CTAB surface between 40 and 100 $m^2/g$.

17. Process according to any one of the claims 1 to 14 of the thickening type, characterized in that the silica has a BET surface between 120 and 450 $m^2/g$ and more particularly between 120 and 200 $m^2/g$.

18. Process according to claim 17, characterized in that the silica has a CTAB surface between 120 and 400 $m^2/g$.

19. Process according to one of the claims 1 to 14 of the bifunctional type, characterized in that the silica has a BET surface between 80 and 200 $m^2/g$.

20. Process according to claim 19, characterized in that the silica has a CTAB surface between 80 and 200 $m^2/g$.

21. Process according to one of the claims 1 to 20, characterized in that the silica has an oil absorption between 80 and 500 $cm^3/100g$.

22. Process according to one of the claims 1 to 21, characterized in that the silica has a mean particle size between 1 and 10 $\mu m$.

23. Process according to one of the claims 1 to 22, characterized in that the silica has an apparent density between 0.01 and 0.3.

24. Process according to one of the claims 1 to 23, characterized in that the silica is a precipitation silica.

25. Process according to one of the claims 1 to 24, characterized in that it consists of preparing the silica gel or suspension by simultaneously adding the silicate and the acid to a sediment, which can be water, a colloidal silica dispersion containing 0 to 150 g/l of silica, expressed in $SiO_2$, a silicate, or an organic or mineral salt, preferably alkali metal salt.

26. Process according to one of the claims 1 to 24, characterized in that the addition of the two reagents takes place simultaneously, so that the pH is kept constant between 4 and 10 and preferably between 8.5 and 9.5.

27. Process according to one of the claims 1 to 25, characterized in that the temperature is between 60 and 95°C.

28. Process according to one of the claims 1 to 24, characterized in that the preparation of the colloidal silica dispersion containing 20 to 150 g/l of silica takes place by heating an aqueous silicate solution between 60 and 95°C and adding the acid to said aqueous solution until a pH is obtained between 8.0 and 10.0 and preferably of 9.5.

29. Process according to one of the claims 1 to 28, characterized in that a first aging of the silica gel or suspension takes place at a pH between 6 and 8.5 and preferably of 8.0 and at a temperature between 60 and 100°C and preferably at 95°C.

30. Process according to one of the claims 1 to 29, characterized in that it consist of preparing a silica gel or suspension by progressively adding the acid to a sediment containing the silicate until the desired aging pH is obtained at a temperature between 60 and 95°C.

31. Process according to one of the claims 1 to 24, characterized in that washing takes place with water or using an acid solution having a temperature between 40 and 80°C and preferably between 60 and 80°C.

32. Process according to claim 31, characterized in that the aforementioned acid solution is a solution of an organic acid, particularly a complexing acid.

33. Process according to claim 32, characterized in that the aforementioned organic acid is chosen from within the group of carboxylic acids, dicarboxylic acids, aminocarboxylic acids and hydroxycarboxylic acids.

34. Process according to one of the claims 32 and 33, characterized in that the organic acid is chosen from within the group including acetic, gluconic, tartaric, citric, maleic and glyceric acids.

35. Dentifrice composition, characterized in that it contains a silica prepared by the process according to one of the claims 1 to 34.

36. Dentifrice composition according to claim 35, characterized in that it comprises at least one element chosen from within the group including fluorine and phosphates.

37. Dentifrice composition according to claim 35, characterized in that it comprises at least one divalent and higher metal cation chosen in group 2a, 3a, 4a and 8 of the periodic classification.

38. Dentifrice composition according to claim 35, characterized in that the metal cation is calcium, strontium, barium, aluminium, indium, germanium, tin, lead, manganese, iron, nickel, zinc, titanium, zirconium or palladium.

39. Dentifrice composition according to one of the claims 35 and and 36, characterized in that the metal cation is in the form of mineral salts chloride, fluoride, nitrate, phosphate, sulphate or in the form of organic salts acetate or citrate.

40. Dentifrice composition according to one of the claims 35 to 39, characterized in that the metal cation is in the form of zinc citrate, zinc sulphate, strontium chloride or tin fluoride.